# EUROPEAN PATENT APPLICATION

(11) **EP 4 714 466 A1**
(43) Date of publication of application: **25.03.2026**
(21) Application number: 24806118.6
(22) Date of filing: 08.02.2024
(51) Int. Cl.: A61K 39/215, C12N 15/113, C12N 15/44, C12N 15/11, A61K 48/00

(54) **DEVELOPMENT OF MULTI-ANTIGEN MRNA VACCINE AGAINST FELINE FIPV**

(30) Priority: 18.05.2023 CN 202310565416
(71) Applicant: Beijing Syngentech Co., Ltd., Beijing 102609 (CN); Jinyu Baoling Biopharmaceutical Co., Ltd., Hohhot, Inner Mongolia 010111 (CN)
(72) Inventor: LIU, Gan, Beijing 102609 (CN); LI, Jie, Hohhot, Inner Mongolia 010111 (CN); LIAO, Weixi, Beijing 102609 (CN); WU, Jinxian, Hohhot, Inner Mongolia 010111 (CN); LIU, Qiang, Beijing 102609 (CN); LI, Cuicui, Beijing 102609 (CN); HUANG, Huiya, Beijing 102609 (CN); WANG, Chen, Hohhot, Inner Mongolia 010111 (CN); ZHAO, Pingping, Hohhot, Inner Mongolia 010111 (CN); JIANG, Xinying, Hohhot, Inner Mongolia 010111 (CN); ZHAO, Yifang, Hohhot, Inner Mongolia 010111 (CN)
(74) Representative: Potter Clarkson
(86) International application number: PCT/CN2024/077001
(87) International publication number: WO 2024/234755

(57) **Abstract**

Provided is a pharmaceutical formulation. The pharmaceutical formulation includes a nucleic acid fragment. The nucleic acid fragment is mRNA and includes at least one of a first nucleic acid fragment, a second nucleic acid fragment, or a third nucleic acid fragment. The first nucleic acid fragment encodes an M protein of feline infectious peritonitis virus; the second nucleic acid fragment encodes an N protein of feline infectious peritonitis virus; the third nucleic acid fragment encodes an S protein, an S_ec protein, or an SII protein of feline infectious peritonitis virus; and the first nucleic acid fragment, the second nucleic acid fragment, and the third nucleic acid fragment are linked or not linked.

## Description

### FIELD

The present disclosure relates to the field of biotechnology, and specifically, to development of a multi-antigen mRNA vaccine against feline FIPV, and more specifically, to a pharmaceutical formulation, an isolated nucleic acid molecule, an expression vector, a recombinant virus, a liposome, a vaccine, a recombinant cell, a method for constructing a feline infectious peritonitis virus vaccine and a use, and a method for preventing or treating a disease caused by feline infectious peritonitis virus infection.

### BACKGROUND

Feline coronavirus (FCoV) belongs to the *Coronaviridae* family. The viruses in the *Coronaviridae* family are characterized by being relatively large, round, enveloped, positive-strand RNA viruses with genomes ranging from 27 kb to 32 kb, which encode the replication polymerase, four structural proteins (S, M, N, and E proteins), and several non-structural proteins. The spike protein (S protein) is one of the important structural proteins of coronaviruses, which is responsible for forming the surface protrusions of these viruses and is also one of the key factors for viral infection. The S protein can bind to receptors on host cells, facilitating the virus to entry the interior of host cells to initiate infection. In addition, the S protein is also an important antigen in many coronavirus vaccines. The SII region of the S protein contains fewer epitopes that mediate antibody-dependent enhancement (ADE) of the immune response. The membrane protein (M protein) is involved in the formation of the particle morphology and the localization of coronaviruses. It is a protein that spans the viral envelope and can interact with other proteins to form the structure of the virion. The nucleocapsid protein (N protein) encapsulates the RNA genome of the virus and is involved in the processes of viral gene replication and transcription. In addition, the N protein can induce a response of the host immune system against the virus. The envelope protein (E protein) is a protein on the envelope of coronaviruses, can interact with the M protein to form the structure of the virion, and is also involved in the processes of viral infection and assembly.

Feline coronavirus (FCoV) can be classified into feline enteric coronavirus (FECV) and feline infectious peritonitis virus (FIPV) according to its biotype or pathogenicity. FECV is highly prevalent, infecting intestinal epithelial cells, and causing no symptoms or only mild diarrhea. FIPV primarily infects feline monocytes and macrophages. It is difficult to distinguish FECV from FIPV based on genomic sequences. Although some studies have suggested that FIPV can be distinguished from FECV by amino acid mutations in the spike protein, these mutations have later been found to be more associated with tissue tropism.

FIPV is typically characterized by the occurrence of pyogranulomatous lesions in various tissues and organs, including the lung, liver, spleen, omentum, and brain. The infection of macrophages and monocytes is considered a key part of the pathogenic mechanism. In the terminal stage of FIPV infection, a significant reduction in T cells in peripheral and lymphoid tissues can be observed, coinciding with the frequent occurrence of hypergammaglobulinemia, which indicates the presence of a severe virus-induced immune dysregulation. Humoral immunity appears to have no protective effect and may lead to "early death syndrome." When S antibodies are present at sub-neutralizing titers, they can enhance infection of target cells by binding to Fc receptors. Researchers have made multiple attempts to develop the FIPV vaccines based on humoral immunity, but most of these attempts have failed. The main reason for the failure resides in the occurrence of antibody-dependent enhancement (ADE) of infection, which prevents antibodies from exerting an effective protective effect. Currently, researchers are attempting to control FIPV infection and clearance through cell-mediated immunity (CMI), but have not yet achieved satisfactory protective effects.

Therefore, there is an urgent need in the art to develop a vaccine against FIPV.

### SUMMARY

The present disclosure is provided by the inventors based on the discovery of the following issues and facts.

At present, there have been no new breakthroughs in the research and development of FIPV vaccines over an extended period, which results in almost all FIPV infections ending in the death of cats.

To this end, in a first aspect of the present disclosure, the present disclosure provides a pharmaceutical formulation. According to an embodiment of the present disclosure, the pharmaceutical formulation includes a nucleic acid fragment. The nucleic acid fragment is mRNA and includes at least one of a first nucleic acid fragment, a second nucleic acid fragment, and a third nucleic acid fragment. The first nucleic acid fragment encodes an M protein of feline infectious peritonitis virus; the second nucleic acid fragment encodes an N protein of feline infectious peritonitis virus; the third nucleic acid fragment encodes an S protein, S_ec protein (S extracellular fragment), or SII protein of feline infectious peritonitis virus; and the first nucleic acid fragment, the second nucleic acid fragment, and the third nucleic acid fragment are linked or not linked. According to an embodiment of the present disclosure, the pharmaceutical formulation, expressing any one or combinations of the M protein, the N protein, the S protein, the S_ec protein, or the SII protein of feline infectious peritonitis viruscan stimulate the cell-mediated immune response in animals.

It should be noted that, in the present disclosure, the M protein, the N protein, the S protein, the S_ec protein, or the SII protein of the wild-type FIPV can also be adaptively modified as desired to reduce the virulence of FIPV, while not affecting its three-dimensional structure and retaining its immunogenicity, so as to prepare a new type of FIPV vaccine. According to the sequence alignment results (Table 1 and Table 2), the M protein has an amino acid sequence with at least 89% homology to SEQ ID NO: 1, and the nucleic acid fragment encoding the M protein has a nucleotide sequence with at least 67% homology to a sequence as set forth in any one of SEQ ID NO: 4 to SEQ ID NO: 7; and the N protein has an amino acid sequence with at least 91% homology to SEQ ID NO: 2, and the nucleic acid fragment encoding the N protein has a nucleotide sequence with at least 70% homology to a sequence as set forth in any one of SEQ ID NO: 8 to SEQ ID NO: 11. The S protein has an amino acid sequence with at least 45% homology to SEQ ID NO: 3, and the nucleic acid fragment encoding the S protein has a nucleotide sequence with at least 51% identity to a sequence as set forth in any one of SEQ ID NO: 12 to SEQ ID NO: 15; the S_ec protein has an amino acid sequence with at least 43% homology to amino acids at sites 1 to 1374 of SEQ ID NO: 3, and the nucleic acid fragment encoding the S_ec protein has a nucleotide sequence with at least 51% identity to nucleotides at sites 1 to 4122 of a sequence as set forth in any one of SEQ ID NO: 12 to SEQ ID NO: 15; and the SII protein has an amino acid sequence with at least 62% homology to amino acids at sites 782 to 1433 of SEQ ID NO: 3, and the nucleic acid fragment encoding the SII protein has a nucleotide sequence with at least 57% identity to nucleotides at sites 2344 to 4299 of a sequence as set forth in any of SEQ ID NO: 12 to SEQ ID NO: 15. The FIPV vaccine is not particularly limited, as long as it can produce the receptor binding domains of the modified M protein, N protein, S protein, S_ec protein, or SII protein of FIPV in the living organism, has immunogenicity and can induce an immune response in the animal. In addition, the pharmaceutical formulation can be used to stimulate an immune response in all animals that can be infected with feline infectious peritonitis virus, including but not limited to a cat.

**Table 1: Protein Sequence Homology of Multiple Sub-strains (%)**

| SEQ ID | Description | Type I | | | | | Type II | | | |
|---|---|---|---|---|---|---|---|---|---|---|
| | | KU-2 | UCD1 | Black | HF 1902 | QS | 79-1146 | SH 2211 | Genbank: GQ152141 | Genbank: MK987175 |
| 1 | M | - | 94.3 | 94.7 | 94.7 | 100.0 | 96.3 | 96.3 | 88.6 | - |
| 2 | N | 92.0 | 91.8 | 91.8 | 91.8 | | 91.8 | 91.5 | 91.5 | - |
| 3 | S | 45.0 | 45.1 | 44.7 | 48.1 | 45.0 | 100.0 | 99.3 | 94.8 | 92.7 |
| 4 | S_ec (extracellular fragment, amino acids at sites 1 to 1374 of S protein) | 43.2 | 43.1 | 42.7 | 46.2 | 43.2 | | 99.3 | 94.7 | 92.6 |
| 5 | SII (amino acids at sites 782 to 1433 of S protein) | 63.2 | 63.3 | 62.4 | 63.6 | 63.0 | | 99.4 | 98.0 | 92.6 |

| | | | | | | | | | | |
|---|---|---|---|---|---|---|---|---|---|---|
| Note: - indicates no data. | | | | | | | | | | |

**Table 2: Nucleic Acid Sequence Homology of Multiple Sub-strains (%)**

| SEQ | Description | Type I | | | | Type II | | | | |
|---|---|---|---|---|---|---|---|---|---|---|
| ID | | KU-2 | UCD1 | Black | HF 1902 | QS | 79-1146 | SH 2211 | Genbank: GQ152141 | Genbank: MK987175 |
| 4 | M wild type | - | 91.7 | 91.7 | 91.7 | 100.0 | 93.3 | 93.2 | 84.8 | - |
| 5 | M2 | - | 72.4 | 71.1 | 71.1 | | 72.2 | 72.2 | 69.0 | - |
| 6 | M3 | - | 71.0 | 70.6 | 70.6 | | 70.6 | 70.6 | 67.5 | - |
| 7 | M6 | - | 73.1 | 72.1 | 72.1 | | 73.2 | 73.5 | 69.6 | - |
| 8 | N wild type | 91.9 | 91.3 | 91.0 | 91.0 | | 90.7 | 90.3 | 90.8 | - |
| 9 | N2 | 70.7 | 70.9 | 70.7 | 70.7 | | 70.8 | 70.7 | 70.5 | - |
| 10 | N3 | 71.8 | 71.8 | 71.7 | 71.7 | | 72.3 | 72.1 | 71.6 | - |
| 11 | N6 | 73.8 | 73.6 | 73.7 | 73.7 | | 73.1 | 72.8 | 72.3 | - |
| 12 | S wild type | 56.9 | 56.7 | 55.7 | 57.9 | 55.9 | 100.0 | 99.5 | 91.6 | 96.7 |
| 13 | S2 | 52.5 | 53.2 | 52.3 | 54.4 | 52.0 | | 72.5 | 71.6 | 71.0 |
| 14 | S3 | 51.2 | 52.2 | 51.9 | 53.5 | 51.6 | | 70.9 | 69.9 | 69.4 |
| 15 | S6 | 53.4 | 53.8 | 53.2 | 54.7 | 53.3 | | 73.6 | 72.2 | 72.2 |
| 12 | S_ec wild type | 55.9 | 55.7 | 54.5 | 56.8 | 55.0 | | 99.5 | 91.6 | 96.7 |
| 13 | S2_ec | 51.7 | 52.3 | 51.4 | 53.6 | 51.2 | | 72.3 | 71.4 | 70.8 |
| 14 | S3_ec | 51.2 | 51.4 | 51.1 | 52.9 | 50.9 | | 70.9 | 69.9 | 69.3 |
| 15 | S6_ec | 52.6 | 53.1 | 52.4 | 53.8 | 52.4 | | 73.5 | 72.2 | 72.1 |
| 12 | SII wild type | 65.0 | 64.4 | 64.2 | 64.3 | 64.4 | | 99.5 | 93.7 | 96.9 |
| 13 | SII2 | 59.6 | 59.0 | 59.6 | 59.4 | 58.9 | | 73.1 | 73.5 | 71.3 |
| 14 | SII3 | 58.5 | 57.5 | 59.1 | 58.1 | 58.2 | | 71.5 | 71.5 | 69.6 |
| 15 | SII6 | 60.0 | 59.1 | 60.3 | 59.6 | 60.1 | | 74.9 | 74.7 | 73.2 |

| | | | | | | | | | | |
|---|---|---|---|---|---|---|---|---|---|---|
| Note: - indicates no data. | | | | | | | | | | |

According to an embodiment of the present disclosure, the above-described pharmaceutical formulation may further include at least one of the following additional technical features:

According to an embodiment of the present disclosure, the first nucleic acid fragment, the second nucleic acid fragment, and the third nucleic acid fragment are not linked. In some embodiments of the present disclosure, the mRNA prepared from the isolated nucleic acid fragment, when used as a vaccine, exhibits a good immune effect against FIPV, and can significantly improve various physiological indicators and the survival rate of the cat.

According to an embodiment of the present disclosure, a mass ratio of the first nucleic acid fragment to the second nucleic acid fragment is 10:1 to 1:10. Optionally, the mass ratio of the first nucleic acid fragment to the second nucleic acid fragment is 1:1, or 1:2, or 1:3, or 1:4, or 1:5, or 1:6, or 1:7, or 1:8, or 1:9, or 1:10, or 10:1, or 9:1, or 8:1, or 7:1, or 6:1, or 5:1, or 4:1, or 3:1, or 2:1.

According to an embodiment of the present disclosure, a mass ratio of the second nucleic acid fragment to the third nucleic acid fragment is 10:1 to 1:10. Optionally, the mass ratio of the second nucleic acid fragment to the third nucleic acid fragment is 1:1, or 1:2, or 1:3, or 1:4, or 1:5, or 1:6, or 1:7, or 1:8, or 1:9, or 1:10, or 10:1, or 9:1, or 8:1, or 7:1, or 6:1, or 5:1, or 4:1, or 3:1, or 2:1.

According to a preferred embodiment of the present disclosure, a mass ratio of the first nucleic acid fragment, the second nucleic acid fragment, and the third nucleic acid fragment is 1:1:1. In some embodiments of the present disclosure, when the three unlinked nucleic acid fragments are in the mass ratio of 1:1:1, the prepared RNA, when used as the vaccine, exhibits a good immune effect against FIPV and can significantly improve various physiological indicators and the survival rate of the cat.

According to a preferred embodiment of the present disclosure, the third nucleic acid fragment is selected from the SII protein. In some embodiments of the present disclosure, the SII protein selected as the third nucleic acid fragment to prepare an mRNA vaccine together with the first nucleic acid fragment and the second nucleic acid fragment has a good immune effect against FIPV and can significantly improve various physiological indicators and the survival rate of the cat.

According to an embodiment of the present disclosure, the first nucleic acid fragment, the second nucleic acid fragment, and the third nucleic acid fragment are linked.

According to an embodiment of the present disclosure, a mass ratio of the first nucleic acid fragment to the second nucleic acid fragment is 10:1 to 1:10. Optionally, the mass ratio of the first nucleic acid fragment to the second nucleic acid fragment is 1:1, or 1:2, or 1:3, or 1:4, or 1:5, or 1:6, or 1:7, or 1:8, or 1:9, or 1:10, or 10:1, or 9:1, or 8:1, or 7:1, or 6:1, or 5:1, or 4:1, or 3:1, or 2:1.

According to an embodiment of the present disclosure, a mass ratio of the second nucleic acid fragment to the third nucleic acid fragment is 10:1 to 1:10. Optionally, the mass ratio of the second nucleic acid fragment to the third nucleic acid fragment is 1:1, or 1:2, or 1:3, or 1:4, or 1:5, or 1:6, or 1:7, or 1:8, or 1:9, or 1:10, or 10:1, or 9:1, or 8:1, or 7:1, or 6:1, or 5:1, or 4:1, or 3:1, or 2:1.

According to a preferred embodiment of the present disclosure, a mass ratio of the first nucleic acid fragment, the second nucleic acid fragment, and the third nucleic acid fragment is 1:1:1.

According to an embodiment of the present disclosure, the first nucleic acid fragment is linked to the second nucleic acid fragment, and the third nucleic acid fragment is not linked to each of the first nucleic acid fragment and the second nucleic acid fragment; or the first nucleic acid fragment is linked to the third nucleic acid fragment, and the second nucleic acid fragment is not linked to each of the first nucleic acid fragment and the third nucleic acid fragment; or the second nucleic acid fragment is linked to the third nucleic acid fragment, and the first nucleic acid fragment is not linked to each of the second nucleic acid fragment and the third nucleic acid fragment.

According to an embodiment of the present disclosure, the 3' end of the first nucleic acid fragment is linked to the 5' end of the second nucleic acid fragment, and the third nucleic acid fragment is not linked to each of the first nucleic acid fragment and the second nucleic acid fragment; or the 3' end of the second nucleic acid fragment is linked to the 5' end of the first nucleic acid fragment, and the third nucleic acid fragment is not linked to each of the first nucleic acid fragment and the second nucleic acid fragment; or the 3' end of the first nucleic acid fragment is linked to the 5' end of the third nucleic acid fragment, and the second nucleic acid fragment is not linked to each of the first nucleic acid fragment and the third nucleic acid fragment; or the 3' end of the third nucleic acid fragment is linked to the 5' end of the first nucleic acid fragment, and the second nucleic acid fragment is not linked to each of the first nucleic acid fragment and the third nucleic acid fragment; or the 3' end of the second nucleic acid fragment is linked to the 5' end of the third nucleic acid fragment, and the first nucleic acid fragment is not linked to each of the second nucleic acid fragment and the third nucleic acid fragment; or the 3' end of the third nucleic acid fragment is linked to the 5' end of the second nucleic acid fragment, and the first nucleic acid fragment is not linked to each of the second nucleic acid fragment and the third nucleic acid fragment.

According to an embodiment of the present disclosure, the 3' end of the first nucleic acid fragment is linked to the 5' end of the second nucleic acid fragment, and the 3' end of the second nucleic acid fragment is linked to the 5' end of the third nucleic acid fragment; or the 3' end of the first nucleic acid fragment is linked to the 5' end of the third nucleic acid fragment, and the 3' end of the third nucleic acid fragment is linked to the 5' end of the second nucleic acid fragment; or the 3' end of the second nucleic acid fragment is linked to the 5' end of the first nucleic acid fragment, and the 3' end of the first nucleic acid fragment is linked to the 5' end of the third nucleic acid fragment; or the 3' end of the second nucleic acid fragment is linked to the 5' end of the third nucleic acid fragment, and the 3' end of the third nucleic acid fragment is linked to the 5' end of the first nucleic acid fragment; or the 3' end of the third nucleic acid fragment is linked to the 5' end of the first nucleic acid fragment, and the 3' end of the first nucleic acid fragment is linked to the 5' end of the second nucleic acid fragment; or the 3' end of the third nucleic acid fragment is linked to the 5' end of the second nucleic acid fragment, and the 3' end of the second nucleic acid fragment is linked to the 5' end of the first nucleic acid fragment.

In an exemplary embodiment of the present disclosure, the connection modes of the M, N, S, S_ec, or SII protein of feline infectious peritonitis virus (FIPV) can be combined arbitrarily.

According to an embodiment of the present disclosure, the M protein has the amino acid sequence as set forth in SEQ ID NO: 1.

According to an embodiment of the present disclosure, the M protein has the amino acid sequence with at least 89% homology to the amino acid sequence as set forth in SEQ ID NO: 1, and an amino acid at site 90 is Y, an amino acid at site 102 is V, an amino acid at site 120 is I, an amino acid at site 144 is A, and an amino acid at site 180 is L.

According to an embodiment of the present disclosure, the M protein has the amino acid sequence as set forth in SEQ ID NO: 1.

According to an embodiment of the present disclosure, the N protein has an amino acid sequence with at least 91% homology to the amino acid sequence as set forth in SEQ ID NO: 2.

According to an embodiment of the present disclosure, the N protein has the amino acid sequence as set forth in SEQ ID NO: 2.

According to an embodiment of the present disclosure, the S protein has an amino acid sequence with at least 45% homology to the amino acid sequence as set forth in SEQ ID NO: 3.

According to an embodiment of the present disclosure, the S protein has an amino acid sequence with at least 45% homology to the amino acid sequence as set forth in SEQ ID NO: 3, and an amino acid at site 515 is V, an amino acid at site 577 is Q, an amino acid at site 1385 is V, an amino acid at site 1386 is V, an amino acid at site 1397 is F, and an amino acid at site 1415 is I.

According to an embodiment of the present disclosure, the S protein has the amino acid sequence as set forth in SEQ ID NO: 3.

According to an embodiment of the present disclosure, the S_ec protein has an amino acid sequence with at least 43% homology to amino acids at sites 1 to 1374 of an amino acid sequence as set forth in SEQ ID NO: 3.

According to an embodiment of the present disclosure, the S_ec protein has the amino acid sequence with at least 43% homology to the amino acids at sites 1 to 1374 of the amino acid sequence as set forth in SEQ ID NO: 3, and an amino acid at site 515 is V and an amino acid at site 577 is Q.

According to an embodiment of the present disclosure, the S_ec protein has the amino acid sequence of sites 1 to 1374 of SEQ ID NO: 3.

According to an embodiment of the present disclosure, the SII protein has an amino acid sequence with at least 62% homology to amino acids at sites 782 to 1433 of the amino acid sequence as set forth in SEQ ID NO: 3.

According to an embodiment of the present disclosure, the SII protein has the amino acid sequence with at least 62% homology to amino acids at sites 782 to 1433 of the amino acid sequence as set forth in SEQ ID NO: 3, and an amino acid at site 1385 is V, an amino acid at site 1386 is V, an amino acid at site 1397 is F, and an amino acid at site 1415 is I.

According to an embodiment of the present disclosure, the SII protein has the amino acid sequence of sites 782 to 1433 of SEQ ID NO: 3.

According to an embodiment of the present disclosure, the first nucleic acid fragment has a nucleotide sequence with at least 67% homology to a sequence as set forth in any one of SEQ ID NO: 4 to SEQ ID NO: 7.

According to an embodiment of the present disclosure, the first nucleic acid fragment has a nucleotide sequence as set forth in any one of SEQ ID NO: 4 to SEQ ID NO: 7.

According to an embodiment of the present disclosure, the second nucleic acid fragment has a nucleotide sequence with at least 70% homology to a sequence as set forth in any one of SEQ ID NO: 8 to SEQ ID NO: 11.

According to an embodiment of the present disclosure, the second nucleic acid fragment has a nucleotide sequence as set forth in any one of SEQ ID NO: 8 to SEQ ID NO: 11.

According to an embodiment of the present disclosure, the third nucleic acid fragment has a nucleotide sequence with at least 51% homology to nucleotides at sites 1 to 4122 of a sequence as set forth in any one of SEQ ID NO: 12 to SEQ ID NO: 15.

According to an embodiment of the present disclosure, the third nucleic acid fragment has a nucleotide sequence of sites 1 to 4122 of a sequence as set forth in any one of SEQ ID NO: 12 to SEQ ID NO: 15.

According to an embodiment of the present disclosure, the third nucleic acid fragment has a nucleotide sequence with at least 57% homology to nucleotides at sites 2344 to 4299 of a sequence as set forth in any one of SEQ ID NO: 12 to SEQ ID NO: 15.

According to an embodiment of the present disclosure, the third nucleic acid fragment has a nucleotide sequence of sites 2344 to 4299 of any one of SEQ ID NO: 12 to SEQ ID NO: 15.

According to an embodiment of the present disclosure, the third nucleic acid fragment has a nucleotide sequence with at least 51% homology to a sequence as set forth in any one of SEQ ID NO: 12 to SEQ ID NO: 15.

According to an embodiment of the present disclosure, the third nucleic acid fragment has a nucleotide sequence as set forth in any one of SEQ ID NO: 12 to SEQ ID NO: 15.

According to an embodiment of the present disclosure, the pharmaceutical formulation further includes a fourth nucleic acid fragment. The fourth nucleic acid fragment encodes a signal peptide sequence of MHC I (major histocompatibility complex I) or a sequence having a similar function to a signal peptide of MHC I. According to an embodiment of the present disclosure, the addition of the signal peptide of MHC I to the N-terminus of an antigen sequence can enable a ribosome to attach to an endoplasmic reticulum membrane and guide the intracellular transport of the protein.

It should be noted that, in the present disclosure, the sequence having the similar function to the signal peptide of MHC I can also enable the ribosome to attach to the endoplasmic reticulum membrane and guide the intracellular transport of the protein.

According to an embodiment of the present disclosure, the signal peptide sequence of MHC I includes no transmembrane region.

According to an embodiment of the present disclosure, the signal peptide sequence of MHC I has the amino acid sequence as set forth in SEQ ID NO: 16.

According to an embodiment of the present disclosure, the fourth nucleic acid fragment has a nucleotide sequence as set forth in any one of SEQ ID NO: 17 to SEQ ID NO: 22.

According to an embodiment of the present disclosure, the fifth nucleic acid fragment is located at the 5' end of the nucleic acid fragment or the 5' end of the nucleic acid molecule.

It should be noted that, the fourth nucleic acid fragment is located upstream of the nucleic acid fragment or the nucleic acid molecule, optionally, at the 5' end of the nucleic acid fragment or the 5' end of the nucleic acid molecule.

According to an embodiment of the present disclosure, the pharmaceutical formulation further includes a fifth nucleic acid fragment. The fifth nucleic acid fragment encodes an MITD (a molecule transport signal of MHC I or a molecule transport signal of major histocompatibility complex I) sequence or a sequence having a similar function to MITD. According to an embodiment of the present disclosure, the addition of the MITD sequence to the C-terminus of the nucleic acid molecule can stimulate the proliferation of CD4⁺ T cells and induce the production of more cytokines.

It should be noted that, in the present disclosure, the sequence having the similar function to MITD can also stimulate the proliferation of the CD4⁺ T cells, induce the production of cytokines, and trigger the immune response in animals.

According to an embodiment of the present disclosure, the MITD sequence has the amino acid sequence as set forth in SEQ ID NO: 23.

According to an embodiment of the present disclosure, the fifth nucleic acid fragment has a nucleotide sequence as set forth in any one of SEQ ID NO: 24 to SEQ ID NO: 32.

According to an embodiment of the present disclosure, the fifth nucleic acid fragment is located at the 3' end of the nucleic acid fragment or the 3' end of the nucleic acid molecule.

It should be noted that, the fifth nucleic acid fragment is located downstream of the nucleic acid fragment or the nucleic acid molecule, optionally at the 3' end of the nucleic acid fragment or the 3' end of the nucleic acid molecule.

According to an embodiment of the present disclosure, the nucleic acid fragment or nucleic acid molecule is a linear molecule.

According to an embodiment of the present disclosure, the nucleic acid fragment or nucleic acid molecule has a nucleotide sequence shown in Table 4.

According to an embodiment of the present disclosure, the pharmaceutical formulation further includes a pharmaceutical carrier. The pharmaceutical carrier includes at least one of a liposome, an exosome, a polymer carrier, a viral vector, or a nanoparticle.

It should be noted that, the pharmaceutical carrier refers to a carrier that does not cause obvious irritation to the subject and does not eliminate the biological activity and properties of the pharmaceutical formulation.

In a second aspect of the present disclosure, the present disclosure provides a method for preparing the pharmaceutical formulation described in the first aspect. According to an embodiment of the present disclosure, the method includes: mixing a first nucleic acid fragment, a second nucleic acid fragment, and a third nucleic acid fragment in a predetermined ratio to obtain the pharmaceutical formulation.

The first nucleic acid fragment encodes an M protein of feline infectious peritonitis virus; the second nucleic acid fragment encodes an N protein of feline infectious peritonitis virus; the third nucleic acid fragment encodes an S protein, an S_ec protein, or an SII protein of feline infectious peritonitis virus; and the nucleic acid fragment is mRNA.

According to an embodiment of the present disclosure, the method can be used to prepare a pharmaceutical formulation for preventing or treating a related disease caused by feline infectious peritonitis virus.

According to an embodiment of the present disclosure, a mass ratio of the first nucleic acid fragment to the second nucleic acid fragment is 10:1 to 1:10. Optionally, the mass ratio of the first nucleic acid fragment to the second nucleic acid fragment is 1:1, or 1:2, or 1:3, or 1:4, or 1:5, or 1:6, or 1:7, or 1:8, or 1:9, or 1:10, or 10:1, or 9:1, or 8:1, or 7:1, or 6:1, or 5:1, or 4:1, or 3:1, or 2:1.

According to an embodiment of the present disclosure, a mass ratio of the second nucleic acid fragment to the third nucleic acid fragment is 10:1 to 1:10. Optionally, the mass ratio of the second nucleic acid fragment to the third nucleic acid fragment is 1:1, or 1:2, or 1:3, or 1:4, or 1:5, or 1:6, or 1:7, or 1:8, or 1:9, or 1:10, or 10:1, or 9:1, or 8:1, or 7:1, or 6:1, or 5:1, or 4:1, or 3:1, or 2:1.

According to an embodiment of the present disclosure, a mass ratio of the first nucleic acid fragment, the second nucleic acid fragment, and the third nucleic acid fragment is 1:1:1.

In a third aspect of the present disclosure, the present disclosure provides an isolated nucleic acid molecule. According to an embodiment of the present disclosure, the nucleic acid molecule includes: a first nucleic acid fragment encoding an M protein of feline infectious peritonitis virus; a second nucleic acid fragment encoding an N protein of feline infectious peritonitis virus; and a third nucleic acid fragment encoding an S protein, S_ec protein, or SII protein of feline infectious peritonitis virus. The first nucleic acid fragment, the second nucleic acid fragment, and the third nucleic acid fragment are linked. The nucleic acid molecule is mRNA. According to an embodiment of the present disclosure, the nucleic acid molecule expressing the M protein, the N protein, the S protein, the S_ec protein, or the SII protein of feline infectious peritonitis virus can stimulate the cell-mediated immune response in animals.

It should be noted that, in the present disclosure, the M protein, the N protein, the S protein, the S_ec protein, or the SII protein of the wild-type FIPV can also be adaptively modified as desired to reduce the virulence of FIPV, while not affecting its three-dimensional structure and retaining its immunogenicity, so as to prepare a new type of FIPV vaccine. According to the sequence alignment results (Table 1 and Table 2), the M protein has an amino acid sequence with at least 89% homology to SEQ ID NO: 1, and the nucleic acid fragment encoding the M protein has a nucleotide sequence with at least 67% homology to a sequence as set forth in any one of SEQ ID NO: 4 to SEQ ID NO: 7; and the N protein has an amino acid sequence with at least 91% homology to SEQ ID NO: 2, and the nucleic acid fragment encoding the N protein has a nucleotide sequence with at least 70% homology to a sequence as set forth in any one of SEQ ID NO: 8 to SEQ ID NO: 11. The S protein has an amino acid sequence with at least 45% homology to SEQ ID NO: 3, and the nucleic acid fragment encoding the S protein has a nucleotide sequence with at least 51% homology to a sequence as set forth in any one of SEQ ID NO: 12 to SEQ ID NO: 15; the S_ec protein has an amino acid sequence with at least 43% homology to amino acids at sites 1 to 1374 of SEQ ID NO: 3, and the nucleic acid fragment encoding the S_ec protein has a nucleotide sequence with at least 51% homology to nucleotides at sites 1 to 4122 of a sequence as set forth in any one of SEQ ID NO: 12 to SEQ ID NO: 15; and the SII protein has an amino acid sequence with at least 62% homology to amino acids at sites 782 to 1433 of SEQ ID NO: 3, and the nucleic acid fragment encoding the SII protein has a nucleotide sequence with at least 57% homology to nucleotides at sites 2344 to 4299 of a sequence as set forth in any of SEQ ID NO: 12 to SEQ ID NO: 15. The FIPV vaccine is not particularly limited, as long as it can produce the receptor binding domains of the modified M protein, N protein, S protein, S_ec protein, or SII protein of FIPV in the living organism, has immunogenicity and can stimulate the living organism to produce a corresponding immune response. In addition, the pharmaceutical formulation can be used to stimulate the immune response of all animals that can be infected with feline infectious peritonitis virus, including but not limited to a cat.

According to an embodiment of the present disclosure, the above-described nucleic acid molecule may further include at least one of the following additional technical features:

According to an embodiment of the present disclosure, the first nucleic acid fragment is linked to the second nucleic acid fragment, and the third nucleic acid fragment is not linked to each of the first nucleic acid fragment and the second nucleic acid fragment; or the first nucleic acid fragment is linked to the third nucleic acid fragment, and the second nucleic acid fragment is not linked to each of the first nucleic acid fragment and the third nucleic acid fragment; or the second nucleic acid fragment is linked to the third nucleic acid fragment, and the first nucleic acid fragment is not linked to each of the second nucleic acid fragment and the third nucleic acid fragment.

According to an embodiment of the present disclosure, the 3' end of the first nucleic acid fragment is linked to the 5' end of the second nucleic acid fragment, and the third nucleic acid fragment is not linked to each of the first nucleic acid fragment and the second nucleic acid fragment; or the 3' end of the second nucleic acid fragment is linked to the 5' end of the first nucleic acid fragment, and the third nucleic acid fragment is not linked to each of the first nucleic acid fragment and the second nucleic acid fragment; or the 3' end of the first nucleic acid fragment is linked to the 5' end of the third nucleic acid fragment, and the second nucleic acid fragment is not linked to each of the first nucleic acid fragment and the third nucleic acid fragment; or the 3' end of the third nucleic acid fragment is linked to the 5' end of the first nucleic acid fragment, and the second nucleic acid fragment is not linked to each of the first nucleic acid fragment and the third nucleic acid fragment; or the 3' end of the second nucleic acid fragment is linked to the 5' end of the third nucleic acid fragment, and the first nucleic acid fragment is not linked to each of the second nucleic acid fragment and the third nucleic acid fragment; or the 3' end of the third nucleic acid fragment is linked to the 5' end of the second nucleic acid fragment, and the first nucleic acid fragment is not linked to each of the second nucleic acid fragment and the third nucleic acid fragment.

According to an embodiment of the present disclosure, the 3' end of the first nucleic acid fragment is linked to the 5' end of the second nucleic acid fragment, and the 3' end of the second nucleic acid fragment is linked to the 5' end of the third nucleic acid fragment; or the 3' end of the first nucleic acid fragment is linked to the 5' end of the third nucleic acid fragment, and the 3' end of the third nucleic acid fragment is linked to the 5' end of the second nucleic acid fragment; or the 3' end of the second nucleic acid fragment is linked to the 5' end of the first nucleic acid fragment, and the 3' end of the first nucleic acid fragment is linked to the 5' end of the third nucleic acid fragment; or the 3' end of the second nucleic acid fragment is linked to the 5' end of the third nucleic acid fragment, and the 3' end of the third nucleic acid fragment is linked to the 5' end of the first nucleic acid fragment; or the 3' end of the third nucleic acid fragment is linked to the 5' end of the first nucleic acid fragment, and the 3' end of the first nucleic acid fragment is linked to the 5' end of the second nucleic acid fragment; or the 3' end of the third nucleic acid fragment is linked to the 5' end of the second nucleic acid fragment, and the 3' end of the second nucleic acid fragment is linked to the 5' end of the first nucleic acid fragment.

In an exemplary embodiment of the present disclosure, the connection modes of the M, N, S, S_ec, or SII protein of feline infectious peritonitis virus (FIPV) can be combined arbitrarily.

According to an embodiment of the present disclosure, the M protein has the amino acid sequence as set forth in SEQ ID NO: 1.

According to an embodiment of the present disclosure, the M protein has the amino acid sequence with at least 89% homology to the amino acid sequence as set forth in SEQ ID NO: 1, and an amino acid at site 90 is Y, an amino acid at site 102 is V, an amino acid at site 120 is I, an amino acid at site 144 is A, and an amino acid at site 180 is L.

According to an embodiment of the present disclosure, the M protein has an amino acid sequence as set forth in SEQ ID NO: 1.

According to an embodiment of the present disclosure, the N protein has an amino acid sequence with at least 91% homology to an amino acid sequence as set forth in SEQ ID NO: 2.

According to an embodiment of the present disclosure, the N protein has the amino acid sequence as set forth in SEQ ID NO: 2.

According to an embodiment of the present disclosure, the S protein has an amino acid sequence with at least 45% homology to the amino acid sequence as set forth in SEQ ID NO: 3.

According to an embodiment of the present disclosure, the S protein has the amino acid sequence with at least 45% homology to the amino acid sequence as set forth in SEQ ID NO: 3, and an amino acid at site 515 is V, an amino acid at site 577 is Q, an amino acid at site 1385 is V, an amino acid at site 1386 is V, an amino acid at site 1397 is F, and an amino acid at site 1415 is I.

According to an embodiment of the present disclosure, the S protein has the amino acid sequence as set forth in SEQ ID NO: 3.

According to an embodiment of the present disclosure, the S_ec protein has an amino acid sequence with at least 43% homology to amino acids at sites 1 to 1374 of an amino acid sequence as set forth in SEQ ID NO: 3.

According to an embodiment of the present disclosure, the S_ec protein has the amino acid sequence with at least 43% homology to the amino acids at sites 1 to 1374 of the amino acid sequence as set forth in SEQ ID NO: 3, and an amino acid at site 515 is V and an amino acid at site 577 is Q.

According to an embodiment of the present disclosure, the S_ec protein has the amino acid sequence of sites 1 to 1374 of SEQ ID NO: 3.

According to an embodiment of the present disclosure, the SII protein has an amino acid sequence with at least 62% homology to amino acids at sites 782 to 1433 of an amino acid sequence as set forth in SEQ ID NO: 3.

According to an embodiment of the present disclosure, the SII protein has the amino acid sequence with at least 62% homology to amino acids at sites 782 to 1433 of the amino acid sequence as set forth in SEQ ID NO: 3, and an amino acid at site 1385 is V, an amino acid at site 1386 is V, an amino acid at site 1397 is F, and an amino acid at site 1415 is I.

According to an embodiment of the present disclosure, the SII protein has the amino acid sequence of sites 782 to 1433 of SEQ ID NO: 3.

According to an embodiment of the present disclosure, the first nucleic acid fragment has a nucleotide sequence with at least 67% homology to a sequence as set forth in any one of SEQ ID NO: 4 to SEQ ID NO: 7.

According to an embodiment of the present disclosure, the second nucleic acid fragment has a nucleotide sequence with at least 70% homology to a sequence as set forth in any one of SEQ ID NO: 8 to SEQ ID NO: 11.

According to an embodiment of the present disclosure, the second nucleic acid fragment has a nucleotide sequence as set forth in any one of SEQ ID NO: 8 to SEQ ID NO: 11.

According to an embodiment of the present disclosure, the third nucleic acid fragment has a nucleotide sequence with at least 51% homology to nucleotides at sites 1 to 4122 of a sequence as set forth in any one of SEQ ID NO: 12 to SEQ ID NO: 15.

According to an embodiment of the present disclosure, the third nucleic acid fragment has a nucleotide sequence of sites 1 to 4122 of a sequence as set forth in any one of SEQ ID NO: 12 to SEQ ID NO: 15.

According to an embodiment of the present disclosure, the third nucleic acid fragment has a nucleotide sequence with at least 57% homology to nucleotides at sites 2344 to 4299 of a sequence as set forth in any one of SEQ ID NO: 12 to SEQ ID NO: 15.

According to an embodiment of the present disclosure, the third nucleic acid fragment has a nucleotide sequence of sites 2344 to 4299 of any one of SEQ ID NO: 12 to SEQ ID NO: 15.

According to an embodiment of the present disclosure, the third nucleic acid fragment has a nucleotide sequence with at least 51% homology to a sequence as set forth in any one of SEQ ID NO: 12 to SEQ ID NO: 15.

According to an embodiment of the present disclosure, the third nucleic acid fragment has a nucleotide sequence as set forth in any one of SEQ ID NO: 12 to SEQ ID NO: 15.

According to an embodiment of the present disclosure, the nucleic acid molecule further includes a fourth nucleic acid fragment. The fourth nucleic acid fragment encodes a signal peptide sequence of MHC I (major histocompatibility complex I) or a sequence having a similar function to a signal peptide of MHC I. According to an embodiment of the present disclosure, the addition of the signal peptide of MHC I to the N-terminus of an antigen sequence can enable a ribosome to attach to an endoplasmic reticulum membrane, thereby guiding the intracellular transport of the protein.

It should be noted that, in the present disclosure, the sequence having the similar function to the signal peptide of MHC I can also enable the ribosome to attach to the endoplasmic reticulum membrane, thereby guiding the intracellular transport of the protein.

According to an embodiment of the present disclosure, the signal peptide sequence of MHC I includes no transmembrane region.

According to an embodiment of the present disclosure, the signal peptide sequence of MHC I has the amino acid sequence as set forth in SEQ ID NO: 16.

According to an embodiment of the present disclosure, the fourth nucleic acid fragment has a nucleotide sequence as set forth in any one of SEQ ID NO: 17 to SEQ ID NO: 22.

According to an embodiment of the present disclosure, the fourth nucleic acid fragment is located at the 5' end of the nucleic acid fragment or the 5' end of the nucleic acid molecule.

It should be noted that, the fourth nucleic acid fragment is located upstream of the nucleic acid fragment or the nucleic acid molecule, optionally, at the 5' end of the nucleic acid fragment or the 5' end of the nucleic acid molecule.

According to an embodiment of the present disclosure, the nucleic acid molecule further includes a fifth nucleic acid fragment. The fifth nucleic acid fragment encodes an MITD (a molecule transport signal of MHC I or a molecule transport signal of major histocompatibility complex I) sequence or a sequence having a similar function to MITD. According to an embodiment of the present disclosure, the addition of the MITD sequence to the C-terminus of the nucleic acid molecule can stimulate the proliferation of CD4⁺ T cells and induce the production of more cytokines.

It should be noted that, in the present disclosure, the sequence having the similar function to MITD can also stimulate the proliferation of the CD4⁺ T cells, induce the production of cytokines, and trigger the immune response in animals.

According to an embodiment of the present disclosure, the MITD sequence has the amino acid sequence as set forth in SEQ ID NO: 23.

According to an embodiment of the present disclosure, the fifth nucleic acid fragment has a nucleotide sequence as set forth in any one of SEQ ID NO: 24 to SEQ ID NO: 32.

According to an embodiment of the present disclosure, the fifth nucleic acid fragment is located at the 3' end of the nucleic acid fragment or the 3' end of the nucleic acid molecule.

It should be noted that, the fifth nucleic acid fragment is located downstream of the nucleic acid fragment or the nucleic acid molecule, optionally at the 3' end of the nucleic acid fragment or the 3' end of the nucleic acid molecule.

According to an embodiment of the present disclosure, the nucleic acid fragment or nucleic acid molecule is a linear molecule.

According to an embodiment of the present disclosure, the nucleic acid fragment or nucleic acid molecule has a nucleotide sequence shown in Table 4.

In a fourth aspect of the present disclosure, the present disclosure provides an expression vector. According to an embodiment of the present disclosure, the expression vector carries the nucleic acid molecule in the third aspect of the present disclosure. According to an embodiment of the present disclosure, the expression vector can be expressed in cells, bacteria, yeasts, or felines.

According to an embodiment of the present disclosure, the above-described expression vector may further include at least one of the following additional technical features.

According to an embodiment of the present disclosure, the expression vector is a non-viral vector.

In a fifth aspect of the present disclosure, the present disclosure provides a recombinant virus. According to an embodiment of the present disclosure, the recombinant virus carries the nucleic acid molecule in the third aspect of the present disclosure. The recombinant virus including the nucleic acid molecule in the third aspect can be propagated in large quantities, and the recombinant virus plays an important role in vaccine research and development.

In a sixth aspect of the present disclosure, the present disclosure provides a liposome. According to an embodiment of the present disclosure, the liposome includes a liposome carrier and a nucleic acid fragment. The nucleic acid fragment is as defined in the first aspect of the present disclosure and the third aspect of the present disclosure. The liposome including the liposome carrier and the nucleic acid fragment plays an important role in improving nucleic acid stability, increasing cellular uptake rate, reducing toxic and side effects, improving delivery efficiency, and other aspects.

In a seventh aspect of the present disclosure, the present disclosure provides a vaccine. According to an embodiment of the present disclosure, the vaccine includes the pharmaceutical formulation in the first aspect of the present disclosure, the nucleic acid molecule in the third aspect of the present disclosure, the expression vector in the fourth aspect of the present disclosure, the recombinant virus in the fifth aspect of the present disclosure, or the liposome in the sixth aspect of the present disclosure. According to an embodiment of the present disclosure, the above-described vaccine can efficiently activate the cell-mediated immune response in animals. In addition, the vaccine contains only proteins capable of activating the cellular immune response, which avoids the occurrence of toxic and side effects and has higher safety.

According to an embodiment of the present disclosure, the above-described vaccine may further include at least one of the following additional technical features.

According to an embodiment of the present disclosure, the vaccine further includes an adjuvant.

According to an embodiment of the present disclosure, the adjuvant includes at least one of a TLR agonist or Mn²⁺.

According to an embodiment of the present disclosure, the TLR agonist includes at least one of CpG, R837, MPLA, or derivatives thereof.

In an eighth aspect of the present disclosure, the present disclosure provides a recombinant cell. According to an embodiment of the present disclosure, the recombinant cell carries a nucleic acid fragment, the nucleic acid molecule in the third aspect of the present disclosure, the expression vector in the fourth aspect of the present disclosure, or the recombinant virus in the fifth aspect of the present disclosure.

The nucleic acid fragment includes at least one of a first nucleic acid fragment, a second nucleic acid fragment, or a third nucleic acid fragment. The first nucleic acid fragment encodes an M protein of feline infectious peritonitis virus; the second nucleic acid fragment encodes an N protein of feline infectious peritonitis virus; the third nucleic acid fragment encodes an S protein, S_ec protein, or SII protein of feline infectious peritonitis virus; and the first nucleic acid fragment, the second nucleic acid fragment, and the third nucleic acid fragment are linked or not linked.

According to an embodiment of the present disclosure, the recombinant cell is used to package a virus carrying the nucleic acid molecule for preparing a nucleic acid vaccine or expressing the M, N, S, S_ec, or SII protein of FIPV, so as to stimulate a strong immune response in the animals.

In a ninth aspect of the present disclosure, the present disclosure provides a method for constructing a feline infectious peritonitis virus vaccine. According to an embodiment of the present disclosure, the method includes: introducing a nucleic acid fragment, the nucleic acid molecule in the third aspect of the present disclosure, the expression vector in the fourth aspect of the present disclosure, or the recombinant virus in the fifth aspect of the present disclosure into a recipient cell.

The nucleic acid fragment includes at least one of a first nucleic acid fragment, a second nucleic acid fragment, or a third nucleic acid fragment. The first nucleic acid fragment encodes an M protein of feline infectious peritonitis virus; the second nucleic acid fragment encodes an N protein of feline infectious peritonitis virus; the third nucleic acid fragment encodes an S protein, S_ec protein, or SII protein of feline infectious peritonitis virus; and the first nucleic acid fragment, the second nucleic acid fragment, and the third nucleic acid fragment are linked or not linked.

The method according to an embodiment of the present disclosure can package the virus carrying the nucleic acid molecule for preparing the nucleic acid vaccine. The method for constructing the feline infectious peritonitis virus vaccine is safe, simple, and efficient.

According to an embodiment of the present disclosure, the above-described method for constructing the feline infectious peritonitis virus vaccine may further include at least one of the following additional technical features:

According to an embodiment of the present disclosure, the method further includes, prior to said introducing into the recipient cell, encapsulating the nucleic acid, the expression vector, or the recombinant virus with an encapsulation carrier.

According to an embodiment of the present disclosure, the encapsulation carrier is selected from at least one of a liposome, exosome, polymer carrier, viral vector, or nanoparticle.

According to an embodiment of the present disclosure, the encapsulation carrier is a nanoparticle. The selection of the nanoparticle for encapsulating mRNA can protect mRNA from degradation, and facilitate the delivery of mRNA into the cell by binding to the cell membrane.

According to a specific embodiment of the present disclosure, the encapsulation carrier is a lipid nanoparticle.

According to an embodiment of the present disclosure, the recipient cell is a CRFK cell, HEK293FT cell, HEK293T cell, BHK cell, or insect cell.

According to an embodiment of the present disclosure, the recipient cell is a CRFK cell. According to an embodiment of the present disclosure, the CFRK cell does not cause an immune rejection response in the subject animal.

In a tenth aspect of the present disclosure, the present disclosure provides use of the pharmaceutical formulation in the first aspect of the present disclosure, the nucleic acid molecule in the third aspect of the present disclosure, the expression vector in the fourth aspect of the present disclosure, the recombinant virus in the fifth aspect of the present disclosure, or the liposome in the sixth aspect of the present disclosure in the manufacture of a medicament or vaccine. According to an embodiment of the present disclosure, the medicament or vaccine is used for preventing or treating a disease related to feline infectious peritonitis virus infection. According to an embodiment of the present disclosure, the medicament or vaccine prepared based on the above-described nucleic acid molecule, expression vector, recombinant virus, or recombinant cell exhibits high safety and can activate the cell-mediated immune response in animals in a short period of time.

In an eleventh aspect of the present disclosure, the present disclosure provides a method for preventing or treating a disease caused by feline infectious peritonitis virus infection. According to an embodiment of the present disclosure, the method includes administering to a subject the pharmaceutical formulation in the first aspect of the present disclosure, the nucleic acid molecule in the third aspect of the present disclosure, the expression vector in the fourth aspect of the present disclosure, the recombinant virus in the fifth aspect of the present disclosure, the liposome in the sixth aspect of the present disclosure, the vaccine in the seventh aspect of the present disclosure, or the recombinant cell in the eighth aspect of the present disclosure. According to an embodiment of the present disclosure, administering an effective dose of the pharmaceutical formulation, nucleic acid molecule, expression vector, recombinant virus, liposome, vaccine, or recombinant cell to the subject infected with FIPV can significantly improve various physiological indicators and a survival rate of the subject. In addition, the above-described treatment method exhibits a good immune effect against all prevalent strains of FIPV.

As used herein, the term "effective dose" refers to an amount that produces a function or activity in subjects and is acceptable to subjects.

The effective amount of the pharmaceutical formulation, nucleic acid molecule, expression vector, recombinant virus, liposome, vaccine, or recombinant cell of the present disclosure can vary with the administration mode, the severity of FIPV infection in the subject, etc. The selection of the preferred effective amount can be determined by a person of ordinary skill in the art based on various factors (e.g., through clinical trials). Such factors include, but are not limited to: pharmacokinetic parameters of the active component such as bioavailability, metabolism, half-life period, etc.; and the severity of the FIPV infection in the subject, a weight of the subject, an immune status of the subject, a route of administration, etc. For example, depending on exigencies of the therapeutic situation, several separate doses can be administered daily, or the dose can be proportionally reduced.

According to an embodiment of the present disclosure, the above-described method may further include at least one of the following technical features.

According to an embodiment of the present disclosure, the subject is selected from a cat.

In a twelfth aspect of the present disclosure, the present disclosure provides use of the pharmaceutical formulation in the first aspect, the nucleic acid molecule in the third aspect, the expression vector in the fourth aspect, the recombinant virus in the fifth aspect, the liposome in the sixth aspect, the vaccine in the seventh aspect, or the recombinant cell in the eighth aspect in the prevention or treatment of a disease caused by feline infectious peritonitis virus infection. According to an embodiment of the present disclosure, administering an effective dose of the pharmaceutical formulation, nucleic acid molecule, expression vector, recombinant virus, liposome, vaccine, or recombinant cell to a subject infected with FIPV can significantly improve various physiological indicators and a survival rate of the subject. In addition, the above-described therapeutic method exhibits a good immune effect against all prevalent strains of FIPV.

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become more apparent and readily understood from the following description of the embodiments taken in conjunction with the accompanying drawings.
FIG. 1 shows the detection results of the expression of the M protein, N protein, and S protein according to Example 1 of the present disclosure.
FIG. 2 shows the detection results of the expression of the target mRNA encapsulated by LNP according to Example 2 of the present disclosure.
FIG. 3 shows the changes in survival rate after viral challenge following immunization with the target mRNA encapsulated by LNP according to Example 2 of the present disclosure.
FIG. 4 shows the detection results of the expression of the target mRNA encapsulated by LNP according to Example 3 of the present disclosure.
FIG. 5 shows the changes in survival rate after viral challenge following immunization with the target mRNA encapsulated by LNP according to Example 3 of the present disclosure.
FIG. 6 shows the changes in survival rate after viral challenge following immunization with the target mRNA encapsulated by LNP according to Example 4 of the present disclosure.
FIG. 7 shows the changes in survival rate after viral challenge following immunization with the target mRNA encapsulated by LNP according to Example 5 of the present disclosure.

### DETAILED DESCRIPTION

Embodiments of the present disclosure will be described in detail below, examples of which are illustrated in the accompanying drawings. The embodiments described below with reference to the drawings are illustrative only, and are intended to explain, and should not be construed as a limitation to the present disclosure.

In addition, terms "first" and "second" are only used for descriptive purposes, and cannot be understood as indicating or implying relative importance or implicitly indicating the number of indicated technical features. Therefore, the features defined with "first" and "second" may explicitly or implicitly include at least one of the features. In the description of the present disclosure, "a plurality of" means at least two, for example, two or three, unless specified otherwise.

It should be noted that, in the present disclosure, the terms "homology" and "identity" both refer to the similarity between amino acid or nucleotide sequences, and there is no specific distinction in their meanings.

### Beneficial Effects

The mRNA vaccine for preventing feline infectious peritonitis described in the present disclosure is prepared by constructing a vector encoding at least one of the M, N, S, S_ec, or SII protein of FIPV, and then preparing the mRNA vaccine for preventing FIPV through the lipid nanoparticle (LNP). The vaccine stimulates a subject to produce the cellular immune response. The vaccine, using mRNA containing the amino acid sequences of the M, N, S, S_ec, or SII of FIPV as its main component, has the advantages such as a simple preparation process, high safety, no toxic and side effects, and industrial producibility; administration of an extremely small dose of the vaccine can achieve a sufficiently protective effect, and it is superior to the existing therapeutic methods in terms of safety and effectiveness.

The sequences involved in the present disclosure are shown in Table 3.

**Table 3**

| SEQ ID NO: | Sequence | Description |
|---|---|---|
| 1 | ERYCAMQNTGSQCINGTDSSCSTCFERGGLIWHLANWNFSWS VILIVFITVLKYGRPQFSWLVYGIKMLIMWLLWPIVLALTIFNAY SEYQVSRYVMFGFSVAGAVVTFALWMMYFVRSIQLYRRTKSW WSFNPETNAILCVNALGRSYVLPLDGTPTGVTLTLLSGNLYAEG FKMAGGLTIEHLPKYVMIATPSRTIVYTLVGKQLKATTATGWAY YVKSKAGDYSTEARTDNLSEHEKLLHMV | M |
| 2 | ATQGQRVNWGDEPSKRRGRSNSRGRKNNTIPLSFFNPIQLEPGS KFWSVCPRDFVPKGIGNKDQQIGYWNRQERYRIVKGQRKELPE RWFFYFLGTGPQADAKFKDKIDGVFWVAKDGAMNKPTTLGTR GTNNESKPLKFDGKIPPQFQLEVNRSRNNSRSGSQSRSVSRNRS | N |
| | QSRGRQQSNNQNNVEDTIVAVLQKLGVTEKQRSRSKSRDRGDS KPRDTTPNNANKHTWKKTAGKGDVTNFYGARSASANFGDSDL VANGNAAKSYPQIAECVPSVSSMLFGSQWSAEDDGDQVKVTL THTYYLPKDDAKTSQFLEQIDAYKRPSQVAKDQRQRKSRSKSA EKKPEELSVTLVEAYTDVFDDTQVEMIDEVTN | |
| 3 | TTNNECIQVNVTQLAGNENLIRDFLFSNFKEEGSVVVGGYYPT EVWYNCSRTARTTAFQYFNNIHAFYFVMEAMENSTGNARGKP LLFHVHGEPVSVIISAYRDDVQQRPLLKHGLVCITKNRHINYEQ FTSNQWNSTCTGADRKIPFSVIPTDNGTKIYGLEWNDDFVTAYI SGRSYHLNINTNWFNNVTLLYSRSSTATWEYSAAYAYQGVSNF TYYKLNNTNGLKTYELCEDYEHCTGYATNVFAPTSGGYIPDGF SFNNWFLLTNSSTFVSGRFVTNQPLLINCLWPVPSFGVAAQEFC FEGAQFSQCNGVSLNNTVDVIRFNLNFTADVQSGMGATVFSLN TTGGVILEISCYSDTVSESSSYSYGEIPFGITDGPRYCYVLYNGTA LKYLGTLPPSVKEIAISKWGHFYINGYNFFSTFPIGCISFNLTTGV SGAFWTIAYTSYTEALVQVENTAIKNVTYCNSHINNIKCSQLTA NLNNGFYPVASSEVGFVNKSVVLLPSFFTYTAVNITIDLGMKLS GYGQPIASTLSNITLPMQDNNTDVYCIRSNQFSVYVHSTCKSSL WDNIFNQDCTDVLEATAVIKTGTCPFSFDKLNNYLTFNKFCLSL SPVGANCKFDVAARTRTNEQVVRSLYVIYEEGDNIVGVPSDNS GLHDLSVLHLDSCTDYNIYGRTGVGIIRRTNSTLLSGLYYTSLSG DLLGFKNVSDGVIYSVTPCDVSAQAAVIDGAIVGAMTSINSELL GLTHWTTTPNFYYYSIYNYTSERTRDTAIDSNDVDCEPVITYSNI GVCKNGALVFINVTHSDGDVQPISTGNVTIPTNFTISVQVEYMQ VYTTPVSIDCARYVCNGNPRCNKLLTQYVSACQTIEQALAMGA RLENMEVDSMLFVSENALKLASVEAFNSTENLDSIYKEWPSIG GSWLGGLKDILPSHNSKRKYGSAIEDLLFDKVVTSGLGTVDED YKRCTGGYDIADLVCAQYYNGIMVLPGVANADKMTMYTASL AGGITLGALGGGAVAIPFAVAVQARLNYVALQTDVLNKNQQILA NAFNQAIGNITQAFGKVNDAIHQTSQGLATVAKALAKVQDVV NTQGQALSHLTVQLQNNFQAISSSISDIYNRLDPPSADAQVDRLI TGRLTALNAFVSQTLTRQAEVRASRQLAKDKVNECVRSQSQRF GFCGNGTHLFSLANAAPNGMIFFHTVLLPTAYETVTAWSGICAS DGDRTFGLVVKDVQLTLFRNLDDKFYLTPRTMYQPRVATSSDF VQIEGCDVLFVNATVIDLPSIIPDYIDINQTVQDILENYRPNWTV PEFTLDIFNATYLNLTGEIDDLEFRSEKLHNTTVELAILIDNINNT LVNLEWLNRIETYVKWPWYVWLLIGLVVVFCIPLLLFCCFSTG CCGCIGCLGSCCHSICSRRQFENYEPIEKVHVH | S |
| 4 | GAACGCTACTGTGCCATGCAAAATACAGGCTCGCAGTGCATT AATGGCACAGATTCATCATGTAGCACCTGTTTTGAACGTGGT GGTCTTATTTGGCATCTGGCTAACTGGAACTTCAGCTGGTCTG TAATATTGATTGTTTTTATAACAGTGTTAAAATATGGAAGACC GCAATTCAGCTGGCTCGTTTATGGCATTAAAATGCTGATCATG TGGCTATTATGGCCTATTGTTCTAGCGCTTACGATTTTTAATGC ATACTCTGAGTACCAAGTTTCCAGATATGTAATGTTCGGCTTT AGTGTTGCAGGTGCAGTTGTAACGTTTGCACTATGGATGATG TATTTTGTGAGATCTATTCAGCTGTATAGACGGACCAAATCAT GGTGGTCTTTTAATCCTGAAACCAATGCGATTCTTTGTGTCAA TGCATTGGGTAGAAGCTATGTACTCCCTCTTGATGGCACTCCT ACAGGTGTTACTCTTACCCTACTTTCAGGAAATCTATACGCTG AAGGTTTTAAAATGGCTGGTGGTCTTACCATCGAGCATTTGC CTAAATATGTCATGATTGCTACGCCTAGTAGAACCATCGTTTA CACATTAGTTGGAAAACAACTAAAGGCAACTACTGCCACTG GATGGGCTTACTATGTAAAATCTAAAGCTGGTGATTACTCAAC AGAAGCACGTACTGATAATTTGAGTGAACATGAAAAATTATT ACATATGGTG | M wild type |
| 5 | GAGAGATACTGTGCCATGCAGAACACAGGCAGCCAGTGCAT CAATGGAACAGACAGCAGCTGCAGCACCTGCTTTGAAAGAG | M2 |
| | GAGGCCTGATCTGGCACCTGGCCAACTGGAACTTCAGCTGG AGTGTGATCCTGATTGTCTTCATTACAGTGCTGAAGTACGGC AGGCCCCAGTTCAGCTGGCTGGTGTACGGCATCAAGATGCTC ATCATGTGGCTGCTGTGGCCCATTGTGTTGGCCCTCACCATCT TCAATGCCTACAGCGAGTACCAGGTGTCCAGATACGTGATGT TCGGCTTCTCAGTGGCAGGAGCCGTGGTGACCTTTGCCCTCT GGATGATGTACTTTGTGAGGTCCATCCAGCTCTACAGAAGAA CAAAGAGCTGGTGGAGCTTCAACCCAGAAACCAACGCCATC CTGTGTGTCAATGCCCTGGGCAGATCCTATGTGCTGCCCCTG GATGGCACCCCCACAGGCGTCACCCTCACCCTCCTGAGCGG CAACCTGTACGCCGAGGGCTTCAAGATGGCCGGCGGCCTGA CAATCGAGCACCTGCCCAAGTATGTGATGATTGCCACCCCCA GCAGGACAATAGTCTACACCCTGGTGGGAAAACAGCTGAAG GCTACCACAGCCACAGGCTGGGCCTACTACGTCAAGAGCAA GGCCGGCGACTACAGCACAGAGGCCAGGACCGACAACCTGT CCGAACATGAAAAACTCCTGCACATGGTC | |
| 6 | GAGAGGTACTGCGCCATGCAGAACACAGGCTCCCAGTGCAT CAACGGAACAGACTCCTCCTGCAGCACCTGCTTCGAGAGAG GAGGCCTCATTTGGCACCTGGCCAACTGGAACTTCAGCTGGT CAGTCATTCTGATAGTCTTCATAACAGTGCTGAAGTACGGCA GGCCCCAGTTCTCCTGGCTCGTGTATGGCATCAAGATGCTGAT CATGTGGCTGCTGTGGCCCATCGTCCTGGCCCTGACCATCTTC AACGCCTACTCAGAGTACCAGGTCAGCAGGTACGTGATGTTC GGCTTCTCCGTGGCCGGAGCAGTGGTGACCTTCGCCCTGTGG ATGATGTACTTCGTGAGGAGCATCCAACTGTACAGGAGGACC AAAAGCTGGTGGTCCTTCAACCCAGAAACCAACGCCATCCT CTGCGTGAACGCCCTGGGCAGGTCCTACGTCCTCCCCCTGGA CGGCACCCCCACCGGGGTCACCCTCACCCTCCTGTCAGGGA ACCTGTACGCTGAGGGCTTCAAGATGGCTGGAGGCCTGACA ATTGAACACCTGCCCAAGTACGTCATGATCGCAACACCCTCC AGAACCATCGTCTACACCCTGGTGGGCAAGCAGCTGAAGGC CACCACCGCCACCGGCTGGGCCTACTACGTCAAGTCCAAGG CCGGCGACTACAGCACCGAGGCCAGGACCGACAACCTCTCA GAGCACGAGAAGCTGCTGCACATGGTG | M3 |
| 7 | GAGAGGTACTGTGCCATGCAGAACACCGGTTCCCAGTGCATC AACGGCACAGACTCCTCCTGCTCCACCTGCTTCGAGAGAGG AGGCCTGATCTGGCACCTGGCAAACTGGAACTTCAGCTGGA GCGTGATCCTGATAGTGTTCATAACCGTCCTGAAGTACGGCA GACCACAGTTCTCATGGCTTGTCTATGGCATCAAGATGCTGAT TATGTGGCTGCTTTGGCCTATCGTCCTGGCCCTGACCATCTTC AACGCCTACTCTGAGTACCAGGTGTCAAGGTATGTCATGTTC GGCTTCTCAGTGGCTGGAGCTGTGGTGACCTTTGCTCTGTGG ATGATGTACTTCGTGAGGTCCATCCAGCTGTACAGGAGGACA AAGTCATGGTGGTCCTTCAACCCAGAAACCAATGCCATCCTG TGCGTCAACGCACTGGGCAGAAGCTACGTCCTACCACTGGA CGGCACTCCTACAGGAGTGACCCTGACCCTGCTGTCAGGCAA TCTGTACGCAGAGGGGTTCAAGATGGCCGGTGGCCTGACCAT CGAGCATCTGCCTAAGTACGTGATGATCGCCACCCCTAGCAG GACAATCGTGTACACCCTGGTGGGAAAGCAGCTAAAGGCGA CCACAGCCACAGGCTGGGCCTACTACGTGAAGTCCAAGGCA GGGGACTATTCAACCGAGGCCAGGACCGACAACCTGTCAGA GCACGAGAAGCTGCTGCACATGGTC | M6 |
| 8 | GCCACACAGGGACAACGCGTCAACTGGGGAGATGAACCTTC CAAAAGACGTGGTCGTTCTAACTCTCGTGGTCGGAAGAATAA CACTATACCTCTTTCATTCTTCAATCCCATCCAACTCGAACCA GGATCAAAATTTTGGAGCGTATGTCCGAGAGATTTTGTTCCC AAGGGAATAGGTAACAAGGATCAACAAATTGGTTATTGGAAT AGACAAGAGCGTTACCGCATTGTCAAAGGTCAGCGTAAGGA | N wild type |
| | ACTTCCTGAGAGGTGGTTTTTCTACTTCTTAGGCACAGGACC TCAAGCTGATGCTAAATTTAAAGACAAGATTGATGGAGTCTT CTGGGTTGCAAAGGATGGTGCCATGAATAAACCAACAACACT TGGCACTCGTGGTACCAACAATGAATCCAAACCACTGAAATT TGATGGTAAGATACCACCGCAATTTCAGCTTGAAGTGAACCG ATCTAGGAACAACTCAAGAAGTGGTTCTCAGTCTAGATCTGT CTCTAGAAACAGGTCTCAATCCAGAGGAAGACAACAATCCA ATAATCAGAATAATGTTGAGGATACAATTGTAGCTGTGCTTCA GAAATTAGGTGTTACTGAAAAGCAAAGGTCACGTTCTAAATC TAGAGATCGTGGTGACTCTAAACCTAGAGACACAACACCTAA TAATGCCAACAAACACACCTGGAAGAAGACTGCAGGTAAAG GTGATGTGACAAATTTCTATGGTGCTAGAAGTGCTTCAGCTA ACTTTGGTGATAGTGATCTCGTTGCCAACGGTAACGCTGCCA AATCCTACCCTCAGATAGCTGAATGCGTTCCATCAGTGTCTAG CATGCTCTTCGGTAGTCAATGGTCTGCTGAAGATGATGGTGAT CAAGTGAAAGTCACGCTCACTCATACCTATTACCTGCCAAAA GATGATGCCAAAACCAGCCAATTCCTAGAACAGATTGACGCT TACAAGCGGCCATCTCAAGTGGCTAAAGATCAGAGGCAAAG AAAATCTCGTTCTAAGTCTGCTGAGAAGAAGCCTGAGGAATT GTCTGTAACTCTTGTAGAGGCATATACGGATGTGTTTGATGAC ACACAGGTTGAGATGATTGATGAGGTTACGAAC | |
| 9 | GCCACACAGGGCCAGAGGGTGAACTGGGGCGACGAGCCATC CAAGAGGAGGGGAAGGAGCAACAGCAGAGGAAGGAAGAA CAACACCATCCCCCTGTCCTTCTTCAACCCAATTCAGCTAGA GCCAGGCAGCAAGTTCTGGTCAGTGTGCCCCAGAGACTTCG TGCCCAAGGGCATCGGAAACAAGGACCAGCAGATCGGCTAC TGGAACAGACAGGAGAGATACAGAATTGTGAAAGGCCAGAG AAAGGAGCTGCCAGAGAGGTGGTTCTTCTACTTCCTGGGCA CCGGCCCACAGGCAGACGCCAAGTTCAAGGACAAGATCGAT GGAGTGTTCTGGGTGGCCAAGGACGGCGCCATGAACAAGCC CACCACACTGGGCACAAGAGGAACAAACAATGAGAGCAAG CCACTGAAGTTTGATGGCAAGATCCCACCCCAGTTCCAGCTG GAGGTGAACAGGAGCAGAAACAACAGCAGAAGCGGCAGCC AGAGCAGAAGTGTGAGCAGAAACAGAAGCCAGAGCAGAGG AAGACAGCAGAGCAACAACCAGAACAACGTGGAGGACACC ATCGTGGCCGTGCTGCAGAAGCTGGGGGTCACAGAAAAGCA GAGGAGCAGAAGCAAGAGCAGGGACAGAGGAGACAGCAA GCCAAGAGACACCACCCCCAACAACGCCAACAAGCACACCT GGAAGAAGACAGCCGGCAAGGGAGATGTGACCAACTTCTAC GGCGCCAGAAGCGCCAGCGCCAACTTCGGAGACTCAGACCT GGTGGCCAATGGAAACGCAGCCAAGAGCTACCCCCAGATCG CAGAGTGTGTGCCCTCTGTCTCCAGCATGCTGTTTGGCAGCC AGTGGAGCGCCGAGGACGACGGTGACCAGGTGAAGGTGAC CCTGACACACACATACTACCTGCCCAAAGATGACGCCAAGAC CAGCCAGTTCCTGGAGCAGATTGATGCCTACAAGAGGCCCA GCCAGGTGGCCAAGGACCAGAGACAGAGGAAGAGCAGGTC CAAGAGCGCCGAGAAGAAGCCAGAAGAATTGAGTGTCACCC TGGTGGAGGCCTACACAGACGTGTTTGATGACACCCAGGTG GAGATGATTGATGAGGTGACCAAC | N2 |
| 10 | GCCACCCAGGGCCAGAGAGTGAACTGGGGCGACGAGCCCTC AAAAAGGAGGGGCAGATCCAACAGCAGAGGCAGGAAGAAC AACACCATCCCCCTGAGCTTCTTCAACCCCATCCAGCTGGAG CCAGGCTCCAAGTTCTGGTCAGTGTGCCCAAGGGACTTCGT GCCCAAGGGCATCGGCAACAAGGACCAGCAGATCGGCTACT GGAACAGGCAGGAGAGATACAGAATCGTGAAGGGCCAGAG GAAGGAACTGCCAGAAAGGTGGTTCTTCTACTTCCTGGGCA CCGGCCCCCAGGCTGACGCCAAGTTCAAAGACAAGATCGAC GGGGTGTTCTGGGTGGCCAAGGACGGCGCCATGAACAAGCC | N3 |
| | AACAACACTGGGCACCAGAGGAACCAACAACGAGAGCAAG CCACTGAAGTTTGACGGCAAGATCCCCCCCCAGTTCCAGCTG GAAGTCAACAGGAGCAGGAACAACAGCAGGTCCGGCTCAC AAAGCAGGAGCGTGTCCAGAAACAGATCCCAGTCAAGAGG AAGACAGCAGTCCAACAACCAGAACAACGTGGAGGACACC ATAGTGGCCGTGCTGCAGAAGCTGGGAGTCACAGAGAAGCA GAGGAGCAGATCCAAGTCCAGGGACAGGGGAGACAGCAAG CCCAGGGACACCACACCCAACAACGCCAACAAGCACACCTG GAAGAAGACAGCCGGCAAGGGAGATGTGACCAACTTCTACG GCGCCAGAAGCGCCTCAGCCAACTTCGGAGACTCAGACCTG GTGGCCAACGGAAACGCCGCCAAGAGCTACCCCCAGATCGC CGAATGTGTCCCCTCAGTGTCCTCCATGCTCTTCGGCTCACA GTGGTCAGCAGAGGACGACGGCGACCAGGTGAAGGTGACC CTGACCCACACCTACTACCTGCCCAAGGACGACGCCAAGAC AAGCCAGTTCCTGGAGCAGATCGACGCCTACAAGAGGCCAT CCCAGGTGGCCAAGGACCAGAGGCAGAGGAAGAGCAGAAG CAAGTCAGCCGAGAAGAAACCAGAGGAGCTGTCAGTCACCC TGGTGGAGGCCTACACCGACGTGTTCGACGACACCCAGGTG GAGATGATCGACGAGGTGACCAAC | |
| 11 | GCAACACAAGGACAGAGAGTAAATTGGGGGGATGAGCCCAG CAAGAGGCGAGGCAGAAGCAACTCAAGAGGGAGAAAAAAC AATACCATCCCACTGTCATTCTTCAACCCCATTCAACTGGAGC CAGGCTCTAAATTCTGGAGTGTATGCCCCAGGGACTTTGTGC CCAAGGGCATAGGGAACAAGGACCAGCAAATAGGATACTGG AACCGGCAGGAGAGATACAGAATTGTCAAGGGTCAGAGAAA GGAGCTGCCAGAGAGATGGTTCTTCTACTTCCTAGGAACAGG CCCACAGGCAGACGCTAAGTTCAAGGATAAGATCGATGGTGT CTTCTGGGTCGCCAAGGATGGTGCAATGAATAAACCAACCAC CCTGGGGACCAGGGGGACAAACAATGAGTCCAAGCCCCTCA AGTTTGATGGCAAAATCCCCCCACAGTTCCAGCTGGAGGTCA ACAGGAGCAGGAACAATAGCCGTTCAGGGTCCCAGTCCAGA TCTGTGTCCAGAAACAGGTCCCAGAGCAGGGGACGGCAGCA GAGTAACAACCAGAATAATGTGGAAGACACCATAGTAGCAGT GCTCCAGAAACTGGGGGTCACAGAAAAACAGAGGAGCAGG TCCAAGTCTAGGGACCGTGGGGACTCTAAGCCAAGGGACAC CACACCCAACAACGCCAACAAGCACACATGGAAAAAAACA GCAGGGAAGGGTGATGTCACCAACTTTTACGGGGCCAGGTC AGCCTCTGCAAACTTCGGGGATAGTGACCTGGTGGCCAACG GCAATGCTGCTAAATCCTACCCTCAGATTGCTGAGTGCGTAC CCTCTGTATCCTCTATGCTCTTTGGCTCACAATGGTCTGCTGA GGATGATGGTGACCAGGTCAAGGTCACCTTGACCCATACCTA CTATCTGCCCAAGGATGATGCAAAAACCAGCCAGTTCCTAGA GCAGATAGATGCCTACAAGAGGCCCAGCCAGGTGGCCAAGG ATCAGAGGCAGAGAAAGAGCAGATCCAAGAGCGCAGAAAA GAAACCAGAGGAGTTATCTGTGACCCTGGTGGAGGCCTACA CAGATGTCTTTGATGATACACAGGTGGAAATGATAGATGAGG TGACTAAC | N6 |
| 12 | ACAACAAATAATGAATGCATACAAGTTAACGTAACACAATTG GCTGGCAATGAAAACCTTATCAGAGATTTTCTGTTTAGTAACT TTAAAGAAGAAGGAAGTGTAGTTGTTGGTGGTTATTACCCTA CAGAGGTGTGGTACAACTGCTCTAGAACAGCTCGAACTACT GCCTTTCAGTATTTTAATAATATACATGCCTTTTATTTTGTTATG GAAGCCATGGAAAATAGCACTGGTAATGCACGTGGTAAACCA TTATTATTTCATGTGCATGGTGAGCCTGTTAGTGTTATTATATC GGCTTATAGGGATGATGTGCAACAAAGGCCCCTTTTAAAACA TGGGTTAGTGTGCATAACTAAAAATCGCCATATTAACTATGAA CAATTCACCTCCAACCAGTGGAATTCCACATGTACGGGTGCT GACAGAAAAATTCCTTTCTCTGTCATACCCACGGACAATGGA | S wild type |
| | ACAAAAATCTATGGTCTTGAGTGGAATGATGACTTTGTTACA GCTTATATTAGTGGTCGTTCTTATCACTTGAACATCAATACTAA TTGGTTTAACAATGTCACACTTTTGTATTCACGCTCAAGCACT GCTACCTGGGAATACAGTGCTGCATATGCTTACCAAGGTGTTT CTAACTTCACTTATTACAAGTTAAATAACACCAATGGTCTAAA AACCTATGAATTATGTGAAGATTATGAACATTGCACTGGCTAT GCTACCAATGTATTTGCTCCGACATCAGGTGGTTACATACCTG ATGGATTTAGTTTTAACAATTGGTTCTTGCTTACAAATAGTTC CACTTTTGTTAGTGGCAGGTTTGTAACAAATCAACCATTATTG ATTAATTGCTTGTGGCCAGTGCCCAGTTTTGGTGTAGCAGCA CAAGAATTTTGTTTTGAAGGTGCACAGTTTAGCCAATGTAAT GGTGTGTCTTTAAATAACACAGTGGATGTTATTAGATTCAACC TTAATTTCACTGCAGATGTACAATCTGGTATGGGTGCTACAGT ATTTTCACTGAATACAACAGGTGGTGTCATTCTTGAAATTTCA TGTTATAGTGACACAGTGAGTGAGTCTAGTTCTTACAGTTATG GTGAAATCCCGTTCGGCATAACTGACGGACCACGATACTGTT ATGTACTTTACAATGGCACAGCTCTTAAATATTTAGGAACATT ACCACCCAGTGTAAAGGAAATTGCTATTAGTAAGTGGGGCCA TTTTTATATTAATGGTTACAATTTCTTTAGCACATTTCCTATTGG TTGTATATCTTTTAATTTAACCACTGGTGTTAGTGGAGCTTTTT GGACAATTGCTTACACATCGTATACTGAAGCATTAGTACAAGT TGAAAACACAGCTATTAAAAATGTGACGTATTGTAACAGTCA CATTAATAACATTAAATGTTCTCAACTTACTGCTAATTTGAATA ATGGATTTTATCCTGTTGCTTCAAGTGAAGTAGGTTTCGTTAA TAAGAGTGTTGTGTTATTACCTAGCTTTTTCACATACACCGCT GTCAATATAACCATTGATCTTGGTATGAAGCTTAGTGGTTATG GTCAACCCATAGCCTCGACACTAAGTAACATCACACTACCAA TGCAGGATAACAATACTGATGTGTACTGTATTCGTTCTAACCA ATTCTCAGTTTATGTTCATTCCACTTGCAAAAGTTCTTTATGG GACAATATTTTTAATCAAGACTGCACGGATGTTTTAGAGGCTA CAGCTGTTATAAAAACTGGTACTTGTCCTTTCTCATTTGATAA ATTGAACAATTACTTGACTTTTAACAAGTTCTGTTTGTCGTTG AGTCCTGTTGGTGCTAATTGCAAGTTTGATGTTGCTGCACGTA CAAGAACCAATGAGCAGGTTGTTAGAAGTCTATATGTAATATA TGAAGAAGGAGACAACATAGTGGGTGTACCGTCTGATAATAG CGGTCTGCACGATTTGTCTGTGCTACACCTAGACTCCTGTAC AGATTACAATATATATGGTAGAACTGGTGTTGGTATTATTAGAC GAACTAACAGTACGCTACTTAGTGGCTTATATTACACATCACT ATCAGGTGATTTGTTAGGCTTTAAAAATGTTAGTGATGGTGTC ATTTATTCTGTGACGCCATGTGATGTAAGCGCACAAGCGGCT GTTATTGATGGTGCCATAGTTGGAGCTATGACTTCCATTAACA GTGAACTGTTAGGTCTAACACATTGGACAACGACACCTAATT TTTATTACTACTCTATATATAATTACACAAGTGAGAGGACTCGT GGCACTGCAATTGACAGTAACGATGTTGATTGTGAACCTGTC ATAACCTATTCTAATATAGGTGTTTGTAAAAATGGTGCTTTGGT TTTTATTAACGTCACACATTCTGACGGAGACGTGCAACCAATT AGCACTGGTAATGTCACGATACCTACAAATTTTACTATATCTG TGCAAGTTGAATACATGCAGGTTTACACTACACCAGTATCAAT AGATTGTGCAAGATACGTTTGTAATGGTAACCCTAGATGTAAC AAATTGTTAACACAATATGTGTCTGCATGTCAAACTATTGAAC AAGCACTTGCAATGGGTGCCAGACTTGAAAACATGGAGGTT GATTCCATGTTGTTTGTCTCGGAAAATGCCCTTAAATTGGCAT CTGTTGAGGCGTTCAATAGTACAGAAAATTTAGATCCTATTTA CAAAGAATGGCCTAGCATAGGTGGTTCTTGGCTAGGAGGTCT AAAAGATATACTACCGTCCCATAATAGCAAACGTAAGTATGGT TCTGCTATAGAAGATTTGCTTTTTGATAAAGTTGTAACATCTG GTTTAGGTACAGTTGATGAAGATTATAAACGTTGTACTGGTGG TTACGACATAGCAGACTTGGTGTGTGCTCAATATTACAATGGC | |
| | ATCATGGTTCTACCAGGTGTAGCTAATGCTGACAAGATGACTA TGTACACAGCATCACTTGCAGGTGGTATAACATTAGGTGCAC TTGGTGGTGGCGCCGTGGCTATACCTTTTGCAGTAGCAGTAC AGGCTAGACTTAATTATGTTGCTCTACAAACTGATGTATTGAA TAAAAACCAACAGATCCTGGCTAATGCTTTCAATCAAGCTATT GGTAACATTACACAGGCTTTTGGTAAGGTTAATGATGCTATAC ATCAAACATCACAAGGTCTTGCCACTGTTGCTAAAGCGTTGG CAAAAGTGCAAGATGTTGTCAACACACAAGGGCAAGCTTTA AGTCACCTTACAGTACAATTGCAAAATAATTTTCAAGCCATTA GTAGTTCTATTAGTGATATTTATAACAGGCTTGACGAACTGAG TGCTGATGCACAAGTTGATAGGCTGATTACAGGTAGACTTAC AGCACTTAATGCATTTGTGTCTCAGACTCTAACCAGACAAGC AGAGGTTAGGGCTAGTAGACAACTTGCCAAAGACAAGGTTA ATGAATGTGTTAGGTCTCAGTCTCAGAGATTCGGATTCTGTGG TAATGGTACACATTTGTTTTCACTAGCAAATGCAGCACCAAAT GGCATGATTTTCTTTCATACAGTACTATTACCAACAGCTTATG AAACTGTAACAGCTTGGTCAGGTATTTGTGCTTCAGATGGCG ATCGCACTTTCGGACTTGTCGTTAAAGATGTGCAGTTGACGT TGTTTCGTAATCTAGATGACAAGTTCTATTTGACCCCCAGAAC TATGTATCAGCCTAGAGTTGCAACTAGTTCTGATTTTGTTCAA ATTGAAGGGTGTGATGTGTTGTTTGTCAACGCGACTGTAATT GATTTGCCTAGTATTATACCTGACTATATTGACATTAATCAAAC TGTTCAAGACATATTAGAAAATTACAGACCAAACTGGACTGT ACCTGAATTTACACTTGATATTTTCAACGCAACCTATTTAAAT CTGACTGGTGAAATTGATGACTTAGAGTTTAGGTCAGAAAAG CTACATAACACTACAGTAGAACTTGCCATTCTCATTGATAACA TTAATAATACATTAGTCAATCTTGAATGGCTCAATAGAATTGA AACTTATGTAAAATGGCCTTGGTATGTGTGGCTACTGATAGGT TTAGTAGTAGTATTTTGCATACCATTACTGCTATTTTGCTGTTTT AGCACAGGTTGTTGTGGATGCATAGGTTGTTTAGGAAGTTGT TGTCACTCTATATGTAGTAGAAGACAATTTGAAAATTATGAAC CAATTGAAAAAGTGCATGTCCACTAA | |
| 13 | ACCACCAACAATGAATGCATCCAGGTGAACGTGACCCAGCT GGCAGGCAATGAAAATTTGATCAGAGACTTCCTGTTCAGCAA CTTCAAGGAGGAGGGCAGTGTAGTGGTGGGAGGCTACTACC CAACAGAGGTGTGGTACAACTGCAGCAGAACAGCCAGAACC ACAGCCTTCCAGTACTTCAACAACATCCACGCCTTCTACTTT GTGATGGAGGCCATGGAAAACAGCACAGGAAATGCCAGAGG AAAACCCCTGCTCTTCCACGTGCACGGAGAGCCCGTGTCAG TCATCATCAGCGCCTACAGAGATGACGTCCAGCAGCGGCCCC TGCTGAAGCATGGACTGGTCTGCATCACCAAGAACAGACAC ATCAACTACGAGCAGTTCACCAGCAACCAGTGGAACAGCAC CTGCACAGGAGCAGACAGAAAAATCCCCTTCAGCGTCATCC CCACAGACAACGGCACCAAAATCTATGGCCTGGAGTGGAAT GATGACTTTGTGACAGCCTATATCAGCGGCAGGAGCTACCAC CTCAACATCAACACCAACTGGTTCAACAACGTCACCCTGCTC TACTCCAGATCCAGCACAGCCACCTGGGAGTACAGCGCCGC CTATGCCTACCAGGGAGTCTCCAACTTCACCTACTACAAACT GAACAACACCAACGGCCTGAAAACCTACGAGCTGTGTGAGG ACTACGAGCACTGCACAGGCTATGCCACAAATGTGTTTGCCC CAACCAGCGGAGGCTACATCCCAGACGGCTTCTCCTTCAACA ACTGGTTCCTCCTCACCAACTCCTCCACATTTGTGAGCGGCA GATTTGTGACCAACCAGCCCCTGCTGATCAACTGCCTGTGGC CCGTGCCCAGCTTTGGAGTGGCAGCCCAGGAGTTCTGCTTCG AGGGAGCCCAGTTCAGCCAGTGCAACGGAGTCAGCCTGAAC AACACAGTGGACGTGATCAGATTCAACCTGAACTTCACAGC AGACGTGCAGAGTGGAATGGGAGCCACCGTCTTCAGCCTGA ACACCACAGGAGGAGTGATCCTGGAGATCAGCTGCTACAGC | S2 |
| | GACACAGTGAGCGAGAGCAGCAGCTACAGCTACGGAGAGAT CCCATTTGGCATCACAGATGGCCCCAGGTACTGCTACGTCCT GTACAATGGAACAGCCCTGAAATACCTGGGCACCCTCCCACC CAGCGTGAAGGAGATCGCCATCAGCAAGTGGGGCCACTTCT ACATCAATGGCTACAACTTCTTCAGCACCTTCCCCATCGGCTG CATCTCCTTCAACCTGACCACAGGAGTGAGCGGGGCCTTCTG GACAATCGCCTACACATCCTACACAGAAGCCCTGGTGCAGGT GGAGAACACAGCCATCAAAAACGTCACCTACTGCAACAGCC ACATCAACAACATCAAGTGCAGCCAGCTGACAGCCAACCTG AACAACGGCTTCTACCCAGTGGCCAGCTCAGAGGTGGGCTT CGTGAACAAGAGCGTGGTGCTCCTGCCCAGCTTCTTCACCTA CACAGCAGTGAACATCACAATTGACCTGGGCATGAAGCTGA GCGGCTACGGCCAGCCAATTGCCAGCACCCTCTCCAACATCA CCCTCCCCATGCAGGACAATAACACAGATGTGTACTGCATCA GATCCAACCAGTTCTCTGTCTACGTGCACAGCACCTGCAAAA GCAGCCTGTGGGACAACATCTTCAACCAGGACTGCACAGAT GTCCTGGAGGCCACAGCCGTGATCAAAACAGGCACCTGCCC CTTCAGCTTTGACAAACTCAACAACTACCTTACATTCAACAA ATTCTGCCTCTCCCTCAGCCCAGTGGGAGCCAACTGCAAGTT TGATGTGGCCGCCAGGACCAGGACAAATGAACAAGTGGTCA GAAGCCTCTACGTCATCTACGAGGAGGGAGACAACATCGTG GGGGTCCCCAGCGACAACAGCGGCCTGCACGACCTGAGTGT GCTCCACCTGGACAGCTGCACAGACTACAACATCTACGGCA GGACTGGGGTGGGCATCATCAGAAGAACCAACAGCACACTG CTGAGTGGCCTGTACTACACCAGCCTGAGTGGAGACTTGCTG GGCTTCAAGAATGTGTCAGATGGGGTGATCTACAGTGTGACC CCCTGTGACGTGTCTGCCCAGGCTGCAGTCATCGACGGAGCC ATCGTGGGAGCCATGACCAGCATTAACAGCGAGCTGCTGGGC CTGACCCACTGGACCACCACCCCCAACTTCTACTACTACTCC ATCTACAACTACACATCAGAAAGAACAAGAGACACAGCCAT CGACAGCAATGACGTGGACTGTGAGCCAGTCATCACCTACA GCAACATCGGAGTGTGCAAGAACGGAGCCCTGGTGTTCATC AACGTGACCCACAGCGACGGAGATGTCCAGCCCATCAGCAC AGGAAATGTGACCATCCCAACCAACTTCACCATCAGCGTCCA GGTGGAATACATGCAGGTGTACACCACCCCAGTGTCCATCGA CTGTGCCAGATACGTGTGCAATGGAAACCCCAGATGCAACAA GCTCCTCACCCAGTACGTGTCAGCCTGCCAGACAATCGAGCA GGCCCTGGCCATGGGAGCCAGGCTCGAGAACATGGAAGTGG ACAGCATGCTGTTTGTCTCAGAGAATGCCCTGAAACTGGCCA GCGTGGAGGCCTTCAACAGCACAGAGAACCTGGACAGCATC TACAAGGAGTGGCCATCAATCGGAGGCAGCTGGCTGGGAGG ACTTAAGGACATCCTGCCAAGCCACAACAGCAAAAGAAAGT ACGGCAGCGCCATTGAGGACCTGCTGTTTGACAAGGTGGTC ACCTCCGGCCTGGGCACAGTGGATGAGGACTACAAGAGATG CACCGGCGGCTATGACATTGCCGACCTGGTGTGTGCCCAGTA CTACAATGGCATCATGGTGCTGCCTGGAGTGGCCAACGCCGA CAAAATGACCATGTACACCGCCTCCCTGGCTGGAGGCATCAC ACTGGGAGCCCTGGGGGGAGGAGCAGTGGCCATCCCCTTTG CAGTGGCTGTGCAGGCCAGACTCAACTACGTGGCCCTGCAG ACAGACGTGCTCAACAAGAACCAGCAGATCCTGGCCAACGC TTTCAACCAGGCTATCGGAAACATCACCCAGGCCTTTGGAAA AGTGAATGATGCCATCCACCAGACCAGCCAGGGCCTGGCCA CAGTGGCCAAGGCCCTGGCCAAGGTGCAGGACGTGGTCAAC ACCCAGGGCCAGGCCCTCAGTCACCTCACAGTACAGCTCCA GAACAACTTCCAGGCAATCTCCTCCTCCATCAGCGACATCTA CAACAGGCTGGACCCCCCAAGCGCTGATGCCCAGGTGGACA GACTGATCACAGGAAGACTCACAGCCCTCAACGCATTTGTGT CCCAGACACTGACCAGGCAGGCAGAGGTCAGGGCCAGCAG | |
| | GCAGCTGGCCAAGGACAAGGTGAATGAGTGCGTGAGGAGCC AGAGCCAGAGATTTGGCTTCTGCGGAAACGGCACCCACCTG TTCAGCCTGGCCAACGCCGCCCCCAACGGCATGATTTTCTTC CACACAGTCCTCCTCCCCACAGCCTACGAAACAGTGACAGC CTGGTCAGGCATCTGTGCCAGCGACGGAGACAGAACCTTTG GCCTGGTGGTGAAGGATGTGCAGCTCACCCTCTTCAGAAAC CTGGATGACAAGTTCTACCTCACCCCAAGAACCATGTACCAG CCCAGAGTGGCCACAAGCAGCGACTTTGTGCAGATTGAGGG CTGTGACGTGCTGTTTGTGAATGCAACAGTGATTGACCTCCC AAGCATCATCCCAGATTACATCGACATCAACCAGACAGTGCA GGACATCCTGGAGAACTACAGGCCCAACTGGACAGTGCCAG AGTTCACCCTGGACATCTTCAACGCCACCTACCTGAACCTGA CAGGAGAAATTGACGACCTGGAGTTCAGATCAGAAAAACTT CACAACACCACCGTGGAGCTTGCCATCCTCATTGACAACATT AACAACACACTGGTCAACCTGGAATGGCTGAACAGAATTGA AACCTACGTGAAGTGGCCCTGGTATGTGTGGCTGCTGATTGG ACTGGTGGTGGTGTTCTGCATCCCACTGCTGCTGTTCTGCTG CTTCAGCACCGGCTGCTGTGGATGCATCGGCTGCTTGGGCAG CTGCTGCCACAGCATCTGCAGCAGGAGGCAGTTTGAGAACT ACGAACCAATTGAAAAAGTGCACGTCCAC | |
| 14 | ACCACCAACAACGAGTGCATCCAGGTGAACGTGACCCAGCT GGCAGGCAACGAGAACCTCATCAGAGACTTCCTCTTCTCCA ACTTCAAGGAGGAGGGCTCAGTGGTGGTCGGCGGCTACTAC CCAACAGAGGTGTGGTACAACTGCTCAAGGACCGCCAGAAC CACAGCCTTCCAGTACTTCAACAACATCCACGCCTTCTACTT CGTGATGGAGGCCATGGAGAACTCCACCGGGAACGCCAGGG GCAAGCCACTACTCTTCCACGTGCACGGAGAGCCAGTGAGC GTGATCATCTCAGCCTACAGGGACGACGTGCAGCAGCGCCC CCTGCTGAAGCATGGACTGGTGTGCATCACCAAGAACAGGC ACATCAACTACGAGCAGTTCACCAGCAACCAGTGGAACAGC ACCTGCACCGGCGCAGACAGGAAGATCCCCTTCTCAGTGATC CCAACAGACAACGGAACCAAAATCTACGGCCTGGAGTGGAA CGACGACTTCGTGACCGCCTACATCAGCGGCAGGTCCTACCA TCTCAACATCAACACCAACTGGTTCAACAACGTCACCCTCCT CTACAGCAGGTCATCCACAGCCACCTGGGAGTACTCAGCTGC CTATGCATACCAGGGAGTCTCCAACTTCACATACTACAAACTC AACAACACCAACGGCCTCAAGACCTACGAGCTGTGTGAGGA CTACGAGCACTGCACCGGCTACGCAACAAACGTCTTCGCCCC AACCTCCGGAGGCTACATCCCAGACGGCTTCTCCTTCAACAA CTGGTTCCTCCTCACAAACAGCTCCACCTTCGTGTCAGGAAG GTTCGTGACCAACCAGCCCCTGCTCATCAACTGCCTCTGGCC CGTCCCCTCCTTCGGAGTGGCCGCCCAGGAGTTCTGCTTCGA GGGAGCCCAGTTCTCCCAGTGCAACGGAGTCTCCCTCAACA ACACCGTGGACGTCATCAGATTCAACCTCAACTTCACAGCAG ACGTCCAGAGCGGCATGGGAGCCACCGTGTTCAGCCTGAAC ACCACAGGAGGAGTGATCCTGGAGATCTCCTGCTACTCAGAC ACAGTGTCAGAGTCCTCCTCCTACAGCTACGGAGAGATCCCA TTCGGCATCACAGACGGCCCCAGATACTGCTACGTGCTGTAC AACGGCACAGCCCTGAAGTACCTGGGCACCCTCCCCCCATC AGTGAAGGAGATCGCCATCAGCAAGTGGGGCCACTTCTACAT CAACGGCTACAACTTCTTCTCCACCTTCCCCATCGGCTGCATC AGCTTCAACCTGACCACCGGAGTGTCCGGAGCCTTCTGGAC CATCGCCTACACATCATACACCGAGGCCCTGGTGCAGGTGGA GAACACAGCCATAAAGAACGTGACCTACTGCAACAGCCACA TCAACAACATCAAGTGCTCCCAGCTGACAGCCAACCTGAAC AACGGCTTCTACCCAGTGGCCTCCAGCGAGGTGGGCTTCGTG AACAAGAGCGTGGTCCTACTCCCCTCCTTCTTCACCTACACA GCAGTCAACATCACAATTGACCTGGGCATGAAGCTGTCCGGC | S3 |
| | TACGGCCAGCCAATCGCCAGCACCCTGTCCAACATCACCCTG CCAATGCAGGACAACAACACCGACGTCTACTGCATCAGAAG CAACCAGTTCTCCGTGTACGTCCACTCCACCTGCAAGTCCTC CCTCTGGGACAACATCTTCAACCAGGACTGCACAGACGTGC TGGAGGCCACAGCTGTGATCAAGACAGGAACCTGCCCTTTC TCATTCGACAAGCTCAACAACTACCTGACCTTCAACAAGTTC TGCCTGAGCCTGTCCCCAGTGGGAGCCAACTGCAAGTTCGA CGTGGCCGCCAGAACCAGGACCAACGAGCAGGTGGTCAGA AGCCTGTACGTCATCTACGAGGAGGGAGACAACATCGTGGG AGTGCCCAGCGACAACTCAGGCCTGCACGACCTGAGCGTGC TGCACCTGGACTCCTGCACAGACTACAACATCTACGGCAGGA CAGGAGTGGGCATCATCAGGAGGACCAACAGCACACTGCTG TCCGGCCTCTACTACACCTCCCTGTCCGGAGACTTGCTGGGA TTCAAGAACGTGTCAGACGGAGTCATCTACAGCGTCACCCCA TGTGACGTGAGCGCCCAGGCAGCAGTGATAGACGGAGCCAT CGTGGGAGCCATGACCTCAATCAACTCAGAACTGCTGGGCCT CACCCACTGGACAACAACACCCAACTTCTACTACTACTCCAT CTACAACTACACATCAGAAAGAACAAGGGACACAGCAATCG ACTCCAACGACGTGGACTGTGAGCCAGTCATCACCTACTCCA ACATCGGCGTGTGCAAGAACGGAGCCCTGGTGTTCATCAAC GTCACCCACTCAGACGGCGACGTCCAGCCAATCTCCACAGG AAACGTCACCATCCCCACCAACTTCACCATCAGCGTGCAGGT GGAGTACATGCAGGTCTACACCACCCCAGTCTCCATCGACTG TGCCAGGTACGTGTGCAACGGCAACCCAAGATGCAACAAAC TGCTGACCCAGTACGTGAGCGCCTGCCAGACCATCGAGCAG GCCCTGGCCATGGGCGCCAGGCTGGAGAACATGGAGGTGGA CAGCATGCTCTTTGTGAGCGAGAACGCCCTGAAGCTTGCCA GCGTGGAGGCCTTCAACAGCACCGAAAACCTGGACTCCATC TACAAAGAGTGGCCCTCCATAGGAGGCTCCTGGCTGGGAGG CCTGAAGGACATCCTCCCATCCCACAACAGCAAAAGAAAGT ACGGCAGCGCCATCGAAGACCTGCTGTTCGACAAGGTGGTC ACCTCAGGACTGGGCACAGTGGACGAGGACTACAAGAGGTG CACCGGAGGCTACGACATCGCAGACCTGGTCTGTGCCCAGTA CTACAACGGCATCATGGTGCTCCCAGGCGTGGCCAACGCCG ACAAGATGACCATGTACACAGCAAGCCTGGCTGGAGGAATC ACACTGGGAGCCCTGGGAGGAGGGGCCGTGGCCATTCCATT CGCCGTGGCCGTGCAGGCCAGACTGAACTACGTGGCCCTGC AGACAGACGTGCTAAACAAGAACCAGCAGATCCTGGCCAAC GCCTTCAACCAGGCCATCGGCAACATCACCCAGGCCTTCGGC AAGGTGAACGACGCAATCCACCAGACATCACAGGGCCTGGC AACAGTGGCCAAGGCCCTGGCCAAGGTCCAGGACGTGGTGA ACACCCAGGGCCAGGCCCTCTCACACCTGACAGTCCAGCTG CAGAACAACTTCCAGGCAATCTCCTCCTCCATCTCAGACATC TACAACAGACTGGACCCCCCCTCAGCCGACGCCCAGGTGGA CAGACTCATCACAGGCAGGCTGACCGCCCTCAACGCCTTCGT GTCCCAGACCCTCACCAGGCAGGCCGAGGTGAGGGCCAGCA GGCAGCTCGCCAAGGACAAGGTGAACGAGTGCGTCAGAAG CCAGAGCCAGAGGTTCGGCTTCTGTGGCAACGGCACCCACC TGTTCTCCCTGGCCAACGCAGCCCCCAACGGCATGATCTTCT TCCACACAGTCCTCCTCCCAACAGCATATGAGACAGTCACCG CCTGGTCAGGAATCTGTGCCTCAGACGGGGACAGAACCTTC GGCCTGGTGGTCAAGGACGTGCAGCTGACACTCTTCAGAAA CCTGGACGACAAATTCTACCTGACCCCCAGGACCATGTACCA GCCAAGGGTGGCCACCTCCTCAGACTTCGTGCAGATCGAGG GCTGTGACGTGCTCTTCGTGAACGCCACCGTCATCGACCTCC CATCCATCATCCCAGACTACATCGACATCAACCAGACAGTGC AGGACATCCTGGAGAACTACCGCCCCAACTGGACCGTGCCA GAGTTCACCCTAGACATATTCAACGCCACCTACCTGAACCTG | |
| | ACAGGAGAAATTGACGACCTGGAGTTCAGATCAGAAAAGCT ACACAACACCACCGTGGAGTTAGCCATCCTCATAGACAACAT TAACAACACCCTCGTCAACCTGGAGTGGCTCAACAGGATTG AAACCTACGTGAAGTGGCCCTGGTACGTCTGGCTCCTCATCG GCCTGGTGGTGGTCTTCTGCATCCCACTGCTGCTGTTCTGCT GCTTCTCCACCGGCTGCTGTGGATGCATCGGCTGCCTGGGCT CATGCTGCCACTCAATCTGCTCAAGGAGGCAGTTTGAAAACT ACGAGCCAATAGAAAAAGTCCACGTCCAC | |
| 15 | ACCACAAATAACGAGTGCATTCAGGTCAACGTCACCCAGCTG GCCGGTAACGAGAACCTAATTAGAGACTTCCTATTCTCGAAC TTTAAAGAGGAAGGCTCTGTGGTGGTCGGAGGTTACTACCCC ACAGAAGTGTGGTACAATTGCTCACGTACAGCCAGGACCAC TGCCTTCCAGTACTTCAACAACATTCATGCCTTCTACTTTGTC ATGGAAGCCATGGAGAACTCCACTGGGAATGCCAGAGGAAA GCCTCTCCTCTTCCATGTCCATGGAGAGCCTGTCTCTGTGATT ATCTCAGCATATAGGGATGATGTGCAGCAGCGGCCGCTGCTT AAGCATGGCCTAGTGTGCATTACTAAGAACCGACATATCAATT ATGAGCAGTTCACCTCCAACCAGTGGAACTCCACATGCACTG GTGCTGATAGGAAGATCCCGTTCAGCGTTATCCCCACCGATAA TGGCACAAAGATTTATGGCCTAGAATGGAACGATGATTTTGTT ACTGCCTACATATCAGGAAGAAGTTACCACTTAAACATTAAC ACCAATTGGTTCAATAATGTTACACTTCTGTACTCTCGCAGCA GTACGGCCACTTGGGAGTATTCGGCTGCATATGCCTACCAAG GTGTAAGCAACTTCACCTACTACAAGCTGAACAATACGAACG GTCTGAAGACTTATGAGCTGTGCGAAGACTACGAGCACTGTA CGGGCTATGCGACAAATGTCTTCGCCCCGACGAGCGGCGGGT ACATACCGGATGGCTTCTCCTTCAACAACTGGTTCCTCCTTAC CAATAGCTCCACTTTCGTATCAGGAAGATTTGTTACGAACCA ACCCCTTCTCATTAACTGTCTGTGGCCAGTGCCCTCCTTCGGA GTAGCTGCTCAAGAGTTCTGTTTCGAGGGTGCACAGTTCAGC CAGTGTAATGGAGTGTCGCTGAACAACACTGTGGACGTGATC AGGTTTAATTTGAACTTCACAGCTGATGTTCAGTCCGGCATG GGCGCGACTGTGTTCAGCCTAAACACCACGGGTGGCGTCATC TTGGAGATTAGTTGTTACTCTGACACTGTGTCAGAGAGCAGC AGTTACTCCTACGGAGAAATTCCTTTCGGCATCACAGACGGT CCCCGGTACTGCTATGTGCTGTACAACGGAACTGCTTTGAAG TACCTGGGGACATTGCCACCTTCTGTGAAGGAAATAGCCATC TCTAAGTGGGGTCACTTTTACATTAACGGCTATAATTTCTTTTC CACTTTCCCAATTGGATGCATTAGCTTCAACCTGACAACAGG TGTGTCTGGAGCCTTCTGGACCATCGCCTATACCTCTTACACA GAGGCTCTAGTACAGGTGGAGAACACAGCTATAAAGAACGT GACGTACTGTAACAGTCACATAAACAATATCAAGTGTTCTCA GTTGACTGCGAACTTAAACAATGGGTTTTATCCAGTGGCGAG CTCGGAGGTGGGGTTTGTAAACAAATCTGTGGTGCTGTTGCC CTCCTTCTTCACGTACACTGCAGTGAACATCACCATTGATTTG GGGATGAAACTGTCCGGCTACGGGCAGCCTATAGCATCTACA CTGAGCAATATCACACTGCCCATGCAGGATAACAATACAGAT GTGTACTGTATCCGCTCAAACCAGTTCTCTGTATACGTGCACA GTACATGCAAGAGCTCGCTATGGGACAACATTTTCAACCAGG ATTGTACTGATGTGCTTGAAGCAACTGCAGTGATCAAAACAG GCACATGCCCGTTCAGCTTTGATAAGCTCAACAACTACCTAA CGTTCAACAAGTTCTGCTTGAGCCTGTCTCCAGTAGGCGCCA ATTGCAAGTTTGACGTTGCAGCGCGAACACGGACAAACGAA CAGGTAGTGCGGTCGCTCTATGTTATCTACGAGGAGGGGGAC AACATAGTCGGGGTTCCATCCGACAACTCAGGTTTGCACGAC CTGAGTGTGCTCCATTTGGACTCATGCACGGATTATAACATCT ACGGGCGCACAGGTGTGGGGATAATACGAAGAACAAACTCT ACGCTATTGAGCGGGCTCTACTACACCTCATTGAGTGGGGAC | S6 |
| | CTGCTAGGGTTCAAGAACGTATCTGACGGTGTGATCTATAGC GTCACACCATGTGACGTATCAGCCCAAGCTGCTGTGATTGAC GGGGCGATTGTGGGGGCTATGACTTCAATTAACAGCGAGCTC CTAGGCCTGACCCACTGGACTACCACCCCAAACTTCTACTAC TACAGCATTTATAACTATACCAGTGAGCGCACCAGGGACACT GCCATTGACAGCAATGACGTCGACTGCGAGCCTGTTATTACC TACAGCAACATCGGTGTTTGTAAGAATGGAGCTCTAGTCTTC ATAAACGTAACGCACTCTGATGGCGATGTTCAACCAATTTCC ACTGGGAACGTAACCATACCCACCAACTTTACTATTTCCGTCC AGGTGGAGTACATGCAAGTATATACCACGCCAGTGTCCATCG ACTGCGCTCGGTATGTGTGCAACGGTAACCCACGCTGCAATA AGCTGCTAACGCAGTACGTCAGCGCCTGCCAGACAATAGAG CAGGCATTGGCAATGGGTGCAAGGCTTGAAAACATGGAGGT GGACTCCATGTTGTTCGTGTCTGAAAACGCTCTTAAACTAGC ATCCGTGGAGGCATTCAACAGTACTGAGAACTTGGACTCTAT CTATAAGGAGTGGCCCTCCATTGGGGGCAGCTGGCTTGGAGG TCTAAAAGACATCCTGCCCAGCCACAACTCCAAGAGGAAGT ACGGGTCCGCTATAGAGGACCTCCTCTTTGACAAGGTTGTTA CTTCTGGTCTTGGCACAGTGGACGAAGACTACAAGAGGTGC ACAGGAGGCTATGATATAGCTGACCTGGTGTGTGCTCAATACT ACAACGGTATAATGGTTCTCCCAGGTGTGGCCAACGCTGACA AGATGACAATGTACACAGCCTCTTTAGCTGGAGGCATTACCC TGGGAGCCCTTGGGGGTGGCGCAGTGGCAATTCCATTTGCCG TTGCGGTGCAGGCCCGACTAAACTATGTCGCACTTCAAACAG ATGTGCTCAACAAGAACCAACAAATACTGGCCAACGCTTTCA ACCAGGCCATTGGTAACATTACGCAGGCATTTGGCAAGGTGA ATGACGCCATCCACCAGACCAGCCAGGGACTTGCCACAGTG GCCAAGGCCTTGGCAAAGGTGCAGGATGTCGTGAACACACA GGGTCAGGCCCTCTCTCATTTGACAGTGCAGCTTCAGAATAA CTTCCAAGCAATCAGTTCAAGCATCAGCGACATCTACAACCG GCTGGACCCCCCATCTGCAGATGCGCAGGTGGACAGGCTAAT CACTGGACGCTTGACGGCACTAAATGCCTTTGTCAGCCAAAC TCTGACCCGGCAAGCAGAGGTGCGGGCCAGTAGACAACTGG CCAAAGACAAGGTCAACGAGTGCGTCAGGTCCCAGTCCCAG CGTTTTGGATTCTGTGGGAACGGGACGCACCTGTTCTCATTA GCCAATGCTGCACCCAATGGCATGATCTTTTTCCATACTGTTC TACTTCCTACTGCCTATGAAACCGTGACCGCTTGGAGCGGCA TCTGCGCATCTGATGGCGATAGGACCTTCGGGCTGGTCGTTA AGGATGTCCAGCTAACGCTGTTCCGGAACTTGGATGACAAGT TCTACCTGACCCCCAGGACCATGTACCAGCCGAGAGTGGCA ACGAGTTCTGACTTCGTGCAAATTGAGGGCTGTGACGTCCTG TTTGTTAATGCAACAGTGATCGATCTGCCCAGTATCATACCAG ATTACATAGACATAAACCAGACAGTCCAGGACATACTGGAGA ATTACAGGCCAAACTGGACCGTACCAGAGTTCACGCTGGACA TATTCAACGCTACGTACCTCAATTTGACTGGGGAAATTGATGA CTTGGAGTTCAGGTCGGAGAAGCTCCACAACACCACTGTGG AGCTGGCCATCCTGATTGACAACATCAACAACACTCTGGTGA ACCTGGAGTGGCTAAATCGCATTGAAACCTATGTCAAGTGGC CTTGGTACGTTTGGCTACTGATCGGACTCGTGGTAGTCTTCTG CATACCACTCCTGCTATTTTGCTGCTTCAGCACAGGGTGCTGT GGCTGCATTGGATGCCTAGGTTCCTGCTGTCACAGTATCTGCA GCAGAAGACAATTCGAGAACTACGAGCCCATAGAAAAGGTC CACGTACAT | |
| 16 | MRFVMSPTVLLLLLGALAAPQTWAGS | MHC I signal peptide |
| 17 | ATGAGATTTGTGATGTCCCCCACTGTCCTGCTGCTGCTCCTGG GAGCCCTGGCAGCCCCCCAGACCTGGGCAGGCAGC | MHC I signal peptide |
| 18 | ATGAGATTTGTGATGAGCCCCACTGTGCTGCTGCTGCTGCTG GGAGCCCTGGCAGCCCCCCAGACCTGGGCTGGCTCC | MHC I signal peptide |
| 19 | ATGCGATTCGTCATGTCACCAACCGTTTTACTATTATTACTAGG AGCATTAGCAGCACCGCAAACATGGGCAGGAAGT | MHC I signal peptide |
| 20 | ATGAGATTCGTGATGTCCCCTACCGTACTACTACTCCTACTTG GCGCACTAGCAGCTCCTCAAACTTGGGCCGGATCC | MHC I signal peptide |
| 21 | ATGAGATTTGTGATGAGCCCCACTGTGCTGCTGCTGCTGCTG GGAGCCCTGGCAGCCCCCCAGACCTGGGCTGGCTCA | MHC I signal peptide |
| 22 | ATGCGTTTTGTAATGTCACCTACTGTACTACTACTACTACTCG GAGCACTAGCAGCACCTCAGACTTGGGCCGGATCA | MHC I signal peptide |
| 23 | FLGIIAGVVVLVVTVVVGAVIWRKKCSGRKGPSYSHAARDDST QGSDSSLMAPKV | MITD |
| 24 | TTCCTGGGCATCATCGCAGGAGTGGTGGTGCTGGTGGTGACC GTGGTGGTGGGGGCTGTAATCTGGAGGAAGAAGTGCTCAGG GAGAAAGGGCCCAAGCTACTCTCACGCCGCCAGGGATGACT CCACACAGGGCTCAGACTCCTCACTGATGGCTCCAAAGGTC | MITD |
| 25 | TTCCTGGGCATCATCGCAGGAGTGGTGGTCCTGGTGGTCACA GTCGTGGTGGGAGCAGTGATCTGGAGGAAGAAGTGCTCAGG AAGGAAGGGCCCATCCTACTCCCACGCCGCCAGGGACGACT CAACCCAGGGCTCAGACAGCTCCCTGATGGCCCCCAAGGTG | MITD |
| 26 | TTCCTGGGCATCATCGCTGGCGTGGTGGTCCTGGTGGTGACT GTGGTGGTCGGAGCCGTCATCTGGAGGAAGAAGTGCAGCGG CAGAAAGGGCCCAAGCTACAGCCACGCCGCCAGAGATGACA GCACCCAGGGCAGCGACAGCAGCCTCATGGCCCCCAAGGTC | MITD |
| 27 | TTCCTGGGCATCATCGCAGGAGTGGTGGTGCTGGTGGTGACA GTGGTGGTGGGAGCAGTGATCTGGAGAAAGAAATGCAGCGG GAGAAAGGGCCCCAGCTACAGCCACGCCGCCAGGGACGAC AGCACCCAGGGCAGCGACAGCAGCCTCATGGCCCCCAAGGT G | MITD |
| 28 | TTCCTGGGCATCATCGCCGGCGTGGTGGTCCTGGTGGTGACC GTGGTGGTCGGAGCAGTGATCTGGAGGAAGAAGTGCTCAGG CAGGAAGGGCCCATCCTACAGCCACGCCGCCAGAGATGACA GCACCCAAGGCTCAGACAGCTCCCTGATGGCCCCCAAGGTG | MITD |
| 29 | TTCCTTGGAATCATAGCTGGGGTCGTTGTCCTCGTAGTGACTG TAGTGGTAGGCGCAGTTATCTGGAGGAAGAAATGCTCGGGG AGGAAAGGGCCCTCTTACAGCCATGCTGCCAGGGATGACTCC ACACAGGGGTCAGATAGCAGCCTCATGGCCCCAAAGGTC | MITD |
| 30 | TTCCTGGGCATCATCGCCGGCGTGGTGGTCCTGGTGGTCACA GTGGTGGTGGGAGCTGTGATCTGGAGAAAGAAGTGCAGCGG CAGGAAGGGCCCAAGCTACAGCCACGCTGCCAGAGATGACT CCACCCAGGGCAGCGACAGCAGCCTGATGGCCCCCAAGGTG | MITD |
| 31 | TTCCTGGGCATAATCGCCGGCGTGGTGGTGCTGGTGGTCACA GTGGTGGTCGGAGCAGTGATCTGGAGGAAGAAGTGCTCAGG GAGGAAGGGCCCATCCTACTCCCACGCCGCCAGGGACGACA GCACCCAGGGCTCAGACTCATCCCTGATGGCCCCCAAGGTG | MITD |
| 32 | TTCCTGGGGATAATCGCAGGAGTGGTTGTTCTAGTGGTGACC GTGGTAGTTGGGGCAGTGATCTGGAGAAAGAAATGCTCTGG CCGTAAGGGACCATCCTACTCCCATGCAGCACGTGATGATTCT ACCCAGGGCAGCGACAGTTCATTGATGGCCCCTAAAGTC | MITD |
| 33 | YPYDVPDYAYPYDVPDYAYPYDVPDYA | HA tag peptide |
| 34 | TACCCCTACGATGTGCCAGACTACGCCTACCCCTACGATGTGC CAGACTACGCCTACCCCTACGATGTGCCAGACTACGCC | HA tag peptide |
| 35 | TACCCCTACGACGTGCCCGACTACGCCTACCCCTACGACGTG CCAGACTACGCCTACCCCTACGACGTGCCAGACTACGCC | HA tag peptide |
| 36 | TACCCATACGATGTTCCAGACTACGCTTACCCATATGACGTGC CAGACTATGCCTACCCCTACGACGTGCCCGACTACGCA | HA tag peptide |
| 37 | HHHHHH | His tag peptide |
| 38 | CACCACCACCACCACCAC | His tag peptide |
| 39 | CATCATCATCATCACCAC | His tag peptide |
| 40 | DYKDDDDKDYKDDDDKDYKDDDDK | Flag tag peptide |
| 41 | GACTACAAAGATGATGATGACAAGGACTACAAAGACGACGA CGACAAAGACTACAAGGACGATGACGACAAG | Flag tag peptide |
| 42 | GACTACAAGGATGATGATGACAAGGACTACAAAGACGACGA CGACAAGGACTACAAGGATGACGATGACAAG | Flag tag peptide |
| 43 | GACTATAAAGACGACGATGACAAAGATTATAAAGATGATGAC GACAAGGACTACAAGGATGATGATGACAAG | Flag tag peptide |
| 44 | GGATAGCTACCAGCTTACACTTGCTTCTGACACAACTGTGTT CGCTATCAACCACACAAACAGACGCC | 5' HBB-UTR |
| 45 | GTGAAGCCGGGGCGATCGTCCTGAGGGCCCGGGGCCTCTTG GGCTCCGGGCACCCGAACTTCCTGATCTTTGAATAAAGTCTG AGTGGGCTGCA | 3' HBA-UTR |
| 46 | RAKRGSGATNFSLLKQAGDVEENPGP | 2A linking peptide |
| 47 | AGAGCCAAGAGAGGCAGCGGAGCCACCAACTTCAGCCTGCT GAAGCAGGCCGGCGACGTGGAGGAGAACCCAGGACCT | 2A linking peptide |
| 48 | GGGGSGGGGSGGGGS | GS linking peptide |
| 49 | GGAGGAGGAGGAAGCGGAGGAGGAGGAAGCGGAGGAGGA GGAAGC | GS linking peptide |

**Table 4: Composition of single antigen sequences involved in the embodiments of the present disclosure**

| Name | SEQ ID NO: | | | |
|---|---|---|---|---|
| | Signal peptide | Tag peptide | Gene | MITD |
| sp-HA-M2-MITD | 17 | 34 | 5 | 26 |
| sp-HA-M3-MITD | 18 | 35 | 6 | 25 |
| sp-HA-M6-MITD | 20 | 36 | 7 | 24 |
| sp-His-N2-MITD | 17 | 38 | 9 | 27 |
| sp-His-N3-MITD | 18 | 38 | 10 | 28 |
| sp-His-N6-MITD | 19 | 39 | 11 | 29 |
| sp-His-N2 | 17 | 38 | 9 | - |
| sp-His-N3 | 18 | 38 | 10 | - |
| sp-His-N6 | 19 | 39 | 11 | - |
| His-N2 | - | 38 | 9 | - |
| His-N3 | - | 38 | 10 | - |
| His-N6 | - | 39 | 11 | - |
| sp-Flag-S2-MITD | 17 | 42 | 13 | 30 |
| sp-Flag-S3-MITD | 21 | 41 | 14 | 31 |
| sp-Flag-S6-MITD | 22 | 43 | 15 | 32 |
| sp-Flag-S2 ec-MITD | 17 | 42 | 13 (nucleotides at positions 1 to 4122) | 30 |
| sp-Flag-S3_ec-MITD | 21 | 41 | 14 (nucleotides at positions 1 to 4122) | 31 |
| sp-Flag-S6_ec-MITD | 22 | 43 | 15 (nucleotides at positions 1 to 4122) | 32 |
| sp-Flag-S2 | 17 | 42 | 13 | - |
| sp-Flag-S3 | 21 | 41 | 14 | - |
| sp-Flag-S6 | 22 | 43 | 15 | - |
| sp-Flag-SII2-MITD | 17 | 42 | 13 (nucleotides at positions 2344 to 4299) | 30 |
| sp-Flag-SII3-MITD | 21 | 41 | 14 (nucleotides at positions 2344 to 4299) | 31 |
| sp-Flag-SII6-MITD | 22 | 43 | 15 (nucleotides at positions 2344 to 4299) | 32 |
| sp-M2-MITD | 17 | - | 5 | 26 |
| sp-M3-MITD | 18 | - | 6 | 25 |
| sp-M6-MITD | 20 | - | 7 | 24 |
| sp-N2-MITD | 17 | - | 9 | 27 |
| sp-N3-MITD | 18 | - | 10 | 28 |
| sp-N6-MITD | 19 | - | 11 | 29 |
| sp-N2 | 17 | - | 9 | - |
| sp-N3 | 18 | - | 10 | - |
| sp-N6 | 19 | - | 11 | - |
| N2 | - | - | 9 | - |
| N3 | - | - | 10 | - |
| N6 | - | - | 11 | - |
| sp-S2-MITD | 17 | - | 13 | 30 |
| sp-S3-MITD | 21 | - | 14 | 31 |
| sp-S6-MITD | 22 | - | 15 | 32 |
| sp-S2_ec-MITD | 17 | - | 13 (nucleotides at positions 1 to 4122) | 30 |
| sp-S3_ec-MITD | 21 | - | 14 (nucleotides at positions 1 to 4122) | 31 |
| sp-S6_ec-MITD | 22 | - | 15 (nucleotides at positions 1 to 4122) | 32 |
| sp-S2 | 17 | - | 13 | - |
| sp-S3 | 21 | - | 14 | - |
| sp-S6 | 22 | - | 15 | - |
| sp-SII2-MITD | 17 | - | 13 (nucleotides at positions 2344 to 4299) | 30 |
| sp-SII3-MITD | 21 | - | 14 (nucleotides at positions 2344 to 4299) | 31 |
| sp-SII6-MITD | 22 | - | 15 (nucleotides at positions 2344 to 4299) | 32 |

**Table 5: Composition of multiple antigen sequences involved in the embodiments of the present disclosure**

| Name | SEQ ID NO: | | | | | | | | |
|---|---|---|---|---|---|---|---|---|---|
| | Signal peptide 1 | Tag peptide 1 | Gene 1 | MITD 1 | Linking peptide | Signal peptide 2 | Tag peptide 2 | Gene 2 | MITD 2 |
| sp-HA-M-MIT D-2A-His-N | 20 | 36 | 7 | 24 | 47 | - | 38 | 9 | - |
| sp-HA-M-GS-N-MITD | 20 | 36 | 7 | - | 49 | - | - | 9 | 24 |
| sp-M-MITD-2 A-N | 20 | - | 7 | 24 | 47 | - | - | 9 | - |
| sp-M-GS-N-M ITD | 20 | - | 7 | - | 49 | - | - | 9 | 24 |

| | | | | | | | | | |
|---|---|---|---|---|---|---|---|---|---|
| Note: "sp" represents a signal peptide. | | | | | | | | | |

It should be noted that, the sequences of the products corresponding to the names in Table 4 and Table 5 are formed by sequentially linking the corresponding sequences in SEQ ID NO:" in a direction of the 5' end to 3' end. For example, in Table 5, the sequence of sp-HA-M-MITD-2A-His-N is formed by linking the signal peptide 1 sequence as set forth in SEQ ID NO: 20, the tag peptide 1 sequence as set forth in SEQ ID NO: 36, the gene 1 sequence as set forth in SEQ ID NO: 7, the MITD 1 sequence as set forth in SEQ ID NO: 24, the linking peptide sequence as set forth in SEQ ID NO: 47, the tag peptide 2 sequence as set forth in SEQ ID NO: 38, and the gene 2 sequence as set forth in SEQ ID NO: 9.

The 3' end of the signal peptide 1 sequence as set forth in in SEQ ID NO: 20 is linked to the 5' end of the tag peptide 1 sequence as set forth in SEQ ID NO: 36, the 3' end of the tag peptide 1 as sequence set forth in SEQ ID NO: 36 is linked to the 5' end of the gene 1 sequence as set forth in SEQ ID NO: 7, the 3' end of the gene 1 sequence as set forth in SEQ ID NO: 7 is linked to the 5' end of the MITD 1 sequence as set forth in SEQ ID NO: 24, the 3' end of the MITD 1 sequence as set forth in SEQ ID NO: 24 is linked to the 5' end of the linking peptide sequence as set forth in SEQ ID NO: 47, the 3' end of the linking peptide sequence as set forth in SEQ ID NO: 47 is linked to the 5' end of the tag peptide 2 sequence as set forth in SEQ ID NO: 38, and the 3' end of the tag peptide 2 sequence as set forth in SEQ ID NO: 38 is linked to the 5' end of the gene 2 sequence as set forth in SEQ ID NO: 9.

The present disclosure is described below with reference to specific examples. It should be noted that these examples are merely illustrative and do not limit the present disclosure in any way. If the specific technologies or conditions are not specified in the examples, they shall be carried out according to the technologies or conditions as described in the literature in the art or according to the product instructions. The reagents and instruments used without indicating the manufacturer are all conventional products that can be purchased commercially.

### Example 1: Selection of Target Sequences

According to this example of the present disclosure, the mRNA sequences were synthesized in vitro and expressed in Crandell Reese feline kidney (CRFK) cells and feline dendritic cells (DCs). Sequences with high expression levels were selected as the final target sequences for vaccine preparation. The specific steps were as follows:
1) Codon optimization was performed on the M, N, and S antigen sequences using a feline universal codon library and a feline mesenteric lymph node codon library, with three optimized sequences generated for each antigen.
2) After sequence synthesis (source: Universal Bio), a vector was constructed. The vector had sequences of the feline-derived 5' HBB-UTR (SEQ ID NO: 44), 3' HBA-UTR (SEQ ID NO: 45) and polyA. The target sequence was inserted between the 5' UTR and 3' UTR.
3) After in vitro transcription into mRNA, the mRNA was transfected into the CRFK cells and the feline dendritic cells (DCs) using a transfection reagent, and sequences with high expression levels in these two feline-derived cell lines were screened.

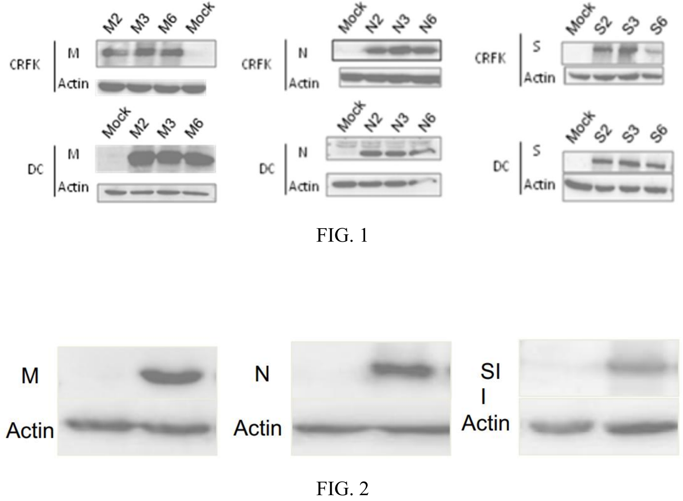

The results, as illustrated in FIG. 1, showed that the target antigen sequences M6 (SEQ ID NO: 7), N2 (SEQ ID NO: 9), and S2 (SEQ ID NO: 13) that can be highly expressed in the two type of cells were successfully screened.

### Example 2: Titer Verification of Vaccine

In this example of the present disclosure, the effectiveness of the vaccine was evaluated by assessing changes in physiological indicators such as body temperature and body weight, as well as survival rate of the subject animal after viral challenge following immunization with the mRNA vaccine.

According to the example of the present disclosure, sp-HA-M6-MITD, sp-His-N2, and sp-SII2-MITD were constructed between the feline-derived 5' HBB-UTR (SEQ ID NO: 44) and 3' HBA-UTR (SEQ ID NO: 45) and transcribed into mRNA in vitro. The sequences were shown in Table 4.

According to the example of the present disclosure, lipid nanoparticles (LNPs) were prepared. The specific steps were as follows:
1) Preparation of lipid solution: The average molecular weight of the liposome system was approximately 620.62. To prepare a 12 mM lipid solution, 42.61 mg of SM-102, 4.52 mg of PEG-DMG, 9.48 mg of DSPC, and 17.86 mg of Chol were weighed, dissolved in 10 mL of anhydrous ethanol, and filter with a 0.22 µm filter membrane.
2) The target mRNA was diluted with a citric acid buffer (pH=4). After mixing thoroughly, a rapid nanodrug preparation system (Mingtai) was used to prepare the mRNA vaccine liposome solution with a pre-conditioned flow rate of 1:3 (volume of organic phase X (containing cationic lipid): volume of aqueous phase Y (containing nucleic acid) = 1:3). Then, the solution was immediately placed in 30 volumes of PBS, concentrated using a 15 mL ultrafiltration tube with a molecular weight cutoff of 100 kDa, and centrifuged at 3000 rpm for 20 minutes. Finally, the concentrate was subjected to equi-volume dilution with 600 mM sucrose solution (prepared in PBS and filtered through a 0.22 µm filter) for storage. The sample was stored at -20°C for later use.

According to this example of the present disclosure, subject animals meeting the test criteria were screened through physical examination and laboratory screening tests. The items of physical examination included body temperature and body weight, and the items of screening tests included PCR assay, N and S protein-binding antibody assay, and neutralizing antibody assay. The specific experimental steps were as follows:
1) Physical Examination: Seven days prior to immunization, the body temperature and body weight of the kittens were measured every day. The normal body temperature was around 38.5°C, and the body weight of a 1-year-old pet cat was approximately 3 kg.
2) Screening of FIPV-negative cats: PCR assay was used to detect the *7ab* gene of FIPV; and the ELISA assay was used to detect binding antibodies in cat serum using the N and S proteins as antigens, and pseudovirus neutralization assay was used to detect neutralization antibodies against FIPV.

According to the example of the present disclosure, the screened subject animals were immunized with the target mRNA encapsulated by LNP. The immunization schedules were as follows:
primary immunization on Day 0 (D0), booster immunization on Day 21 (D21), and viral challenge on Day 28 (D28) after the booster immunization; the challenge strain was QS-1146; and 5 kittens per group.

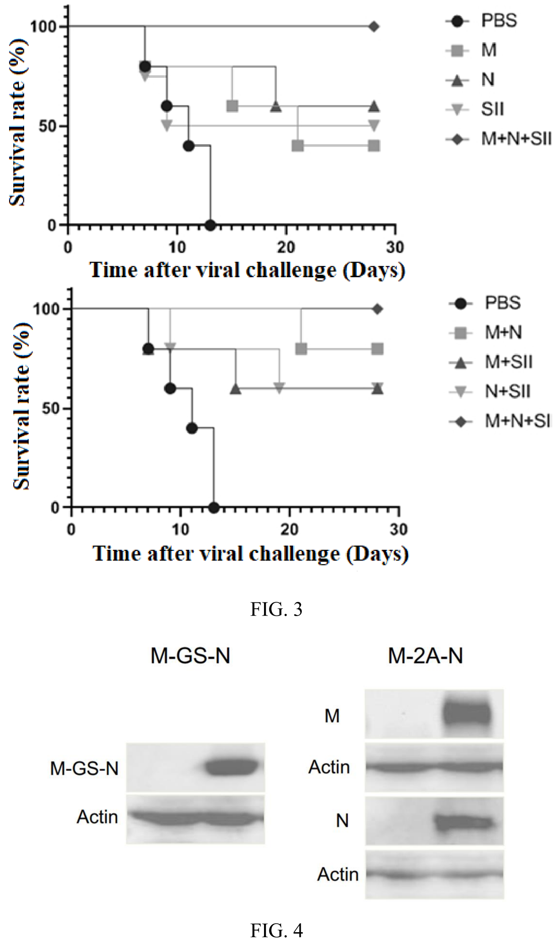

The target mRNA encapsulated by LNP was transfected into the CRFK cells. The transfected CRFK cells were collected after 24 hours, and subjected to a Western blotting assay to detect protein expression. The results, as illustrated in FIG. 2, showed that all mRNAs were normally expressed. After immunization and viral challenge with the target mRNA encapsulated by LNP in each group, all five kittens in the PBS group successively developed symptoms of fever and weight loss. Post-mortem dissection revealed that these kittens had typical pathological lesions of feline infectious peritonitis. In contrast, the kittens in the immunized groups (immunized with mRNA expressing M, N, or SII antigens, or a combination thereof) showed significantly improved physiological indicators, and all kittens in the M+N+SII group remained normal. The survival rate, as illustrated in FIG. 3, showed that immunization with mRNA expressing the individual M, N, and SII antigens improved the survival rate following viral challenge. In addition, the combination of multiple antigens can further improve the survival rate after viral challenge, and the survival rate of the M+N+SII group reached 100% after viral challenge.

The above results showed that mRNAs expressing the individual M, N, and SII antigens or a combination thereof, when used as the vaccine, exhibited a good immune effect against FIPV, significantly improving various physiological indicators and the survival rate, with the combination of M+N+SII yielding the best effect.

### Example 3: Validation of the Titer of Multi-Antigen mRNA Vaccines with Different Linkage Modes

In this example of the present disclosure, the effectiveness of multi-antigen mRNA vaccines with different linkage modes was evaluated. The methods for LNP preparation and expression verification, the method for screening subject animals, immunization procedure, and evaluation method were the same as those in Example 2.

According to the example of the present disclosure, sp-HA-M6-MITD, sp-His-N2, sp-HA-M-MITD-2A-His-N, and sp-HA-M-GS-N-MITD were constructed between the feline-derived 5' HBB-UTR (SEQ ID NO: 44) and 3' HBA-UTR (SEQ ID NO: 45) and transcribed into mRNA in vitro. The sequences were shown in Table 4 and Table 5.

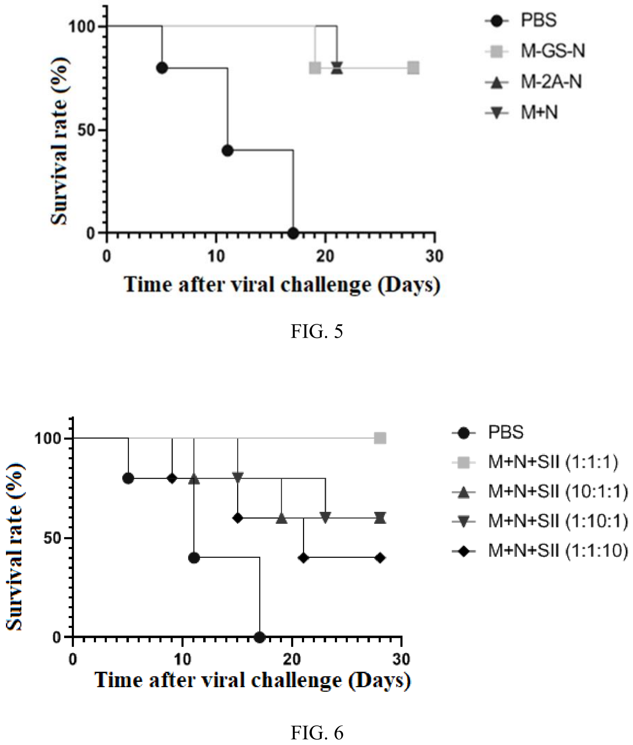

The expression detection results of the target mRNA after encapsulated by LNP were shown in FIG. 4, and all mRNAs were normally expressed. After immunization and viral challenge, the multi-antigen mRNA vaccines with different linkage modes all effectively improved the physiological indicators and survival rate of the subject animals after the viral challenge. The survival rate was shown in FIG. 5, and there were no significant differences between the different linkage modes.

The above results showed that the isolated single-antigen mRNA vaccines (serving as a pharmaceutical composition) and the multi-antigen mRNA vaccines linked by different linking peptides both exhibited good immune effects against FIPV.

### Example 4: Verification of the Titer of Multi-Antigen mRNA Vaccines with Different Ratios

In this example of the present disclosure, the effectiveness of multi-antigen mRNA vaccines at different ratios was evaluated. The method for LNP preparation, the method for screening subject animals, the immunization procedure, and the evaluation method were the same as those in Example 2.

According to the example of the present disclosure, sp-HA-M6-MITD, sp-His-N2, and sp-SII2-MITD were constructed between the feline-derived 5' HBB-UTR (SEQ ID NO: 44) and 3' HBA-UTR (SEQ ID NO: 45) and transcribed into mRNA in vitro. The sequences were shown in Table 4.

After immunization and viral challenge, the multi-antigen mRNA vaccines with different ratios all effectively improved the physiological indicators and survival rate of the subject animals. The survival rate was shown in FIG. 6.

The above results showed that multi-antigen mRNA vaccines with different ratios can all exhibit good immune effects against FIPV.

### Example 5: Verification of the Titer of Multi-Antigen mRNA Vaccines Against Different Prevalent Strains

In this example of the present disclosure, the effectiveness of the multi-antigen mRNA vaccine against different prevalent strains of FIPV was evaluated. The method for LNP preparation, the method for screening subject animals, the immunization schedule, and evaluation method were the same as those in Example 2. The challenge strains were HF1902 and SH2211.

According to the example of the present disclosure, sp-HA-M6-MITD, sp-His-N2, and sp-SII2-MITD were constructed between the feline-derived 5' HBB-UTR (SEQ ID NO: 44) and 3' HBA-UTR (SEQ ID NO: 45) and transcribed into mRNA in vitro. The sequences were shown in Table 4.

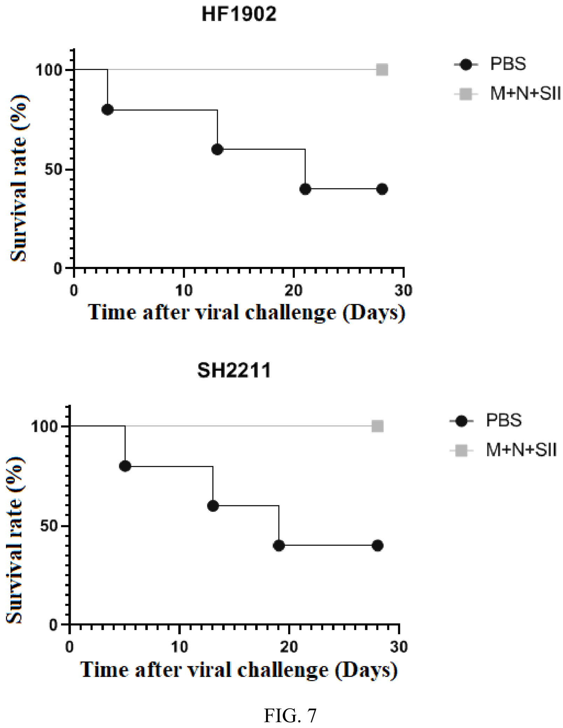

After immunization and viral challenge, the multi-antigen mRNA vaccine can effectively improve the physiological indicators and survival rate of the subject animals against different prevalent strains of FIPV. The survival rate was shown in FIG. 7.

The above results showed that the multi-antigen mRNA vaccine exhibited good immune effects against different prevalent strains of FIPV.

In the description of the present specification, reference to the terms such as "an embodiment," "some embodiments," "an example," "a specific example," or "some examples" mean that a particular feature, structure, material, or characteristic described in connection with the embodiment or example is included in at least one embodiment or example of the present disclosure. In the present specification, the illustrative expressions of the above terms do not necessarily refer to the same embodiment or example. Furthermore, the particular features, structures, materials, or characteristics described may be combined in any suitable manner in one or more embodiments or examples. In addition, without mutual contradiction, those skilled in the art can combine and integrate the different embodiments or examples described in this specification, as well as the features of different embodiments or examples.

Although the embodiments of the present disclosure have been shown and described above, it should be understood that the above-described embodiments are illustrative and should not be construed as limiting the present disclosure. Those skilled in the art can make changes, modifications, substitutions, and variations to the above-described embodiments within the scope of the present disclosure.

## Claims

1. A pharmaceutical formulation, comprising a nucleic acid fragment, the nucleic acid fragment being mRNA and comprising at least one of a first nucleic acid fragment, a second nucleic acid fragment, or a third nucleic acid fragment, wherein:
the first nucleic acid fragment encodes an M protein of feline infectious peritonitis virus;
the second nucleic acid fragment encodes an N protein of feline infectious peritonitis virus;
the third nucleic acid fragment encodes an S protein, S_ec protein, or SII protein of feline infectious peritonitis virus; and
the first nucleic acid fragment, the second nucleic acid fragment, and the third nucleic acid fragment are linked or not linked.

2. The pharmaceutical formulation according to claim 1, wherein the first nucleic acid fragment, the second nucleic acid fragment, and the third nucleic acid fragment are not linked.

3. The pharmaceutical formulation according to claim 1, wherein the first nucleic acid fragment, the second nucleic acid fragment, and the third nucleic acid fragment are linked.

4. The pharmaceutical formulation according to claim 2 or 3, wherein a mass ratio of the first nucleic acid fragment to the second nucleic acid fragment is 10:1 to 1:10.

5. The pharmaceutical formulation according to claim 4, wherein a mass ratio of the second nucleic acid fragment to the third nucleic acid fragment is 10:1 to 1:10.

6. The pharmaceutical formulation according to claim 5, wherein a mass ratio of the first nucleic acid fragment, the second nucleic acid fragment, and the third nucleic acid fragment is 1:1:1.

7. The pharmaceutical formulation according to claim 1, wherein the third nucleic acid fragment is selected from the SII protein.

8. A method for preparing the pharmaceutical formulation according to any one of claims 1 to 7, the method comprising:
mixing a first nucleic acid fragment, a second nucleic acid fragment, and a third nucleic acid fragment in a predetermined ratio to obtain the pharmaceutical formulation, wherein:
the first nucleic acid fragment encodes an M protein of feline infectious peritonitis virus;
the second nucleic acid fragment encodes an N protein of feline infectious peritonitis virus;
the third nucleic acid fragment encodes an S protein, S_ec protein, or SII protein of feline infectious peritonitis virus; and
the first nucleic acid fragment, the second nucleic acid fragment, and the third nucleic acid fragment are each mRNA.

9. The method according to claim 8, wherein a mass ratio of the first nucleic acid fragment to the second nucleic acid fragment is 10:1 to 1:10.

10. The method according to claim 9, wherein a mass ratio of the second nucleic acid fragment to the third nucleic acid fragment is 10:1 to 1:10.

11. The method according to claim 10, wherein a mass ratio of the first nucleic acid fragment, the second nucleic acid fragment, and the third nucleic acid fragment is 1:1:1.

12. An isolated nucleic acid molecule, comprising:
a first nucleic acid fragment encoding an M protein of feline infectious peritonitis virus;
a second nucleic acid fragment encoding an N protein of feline infectious peritonitis virus; and
a third nucleic acid fragment encoding an S protein, S_ec protein, or SII protein of feline infectious peritonitis virus,
wherein the first nucleic acid fragment, the second nucleic acid fragment, and the third nucleic acid fragment are linked; and
wherein the nucleic acid molecule is mRNA.

13. The pharmaceutical formulation according to claim 2 or the nucleic acid molecule according to claim 12, wherein:
the first nucleic acid fragment is linked to the second nucleic acid fragment, and the third nucleic acid fragment is not linked to each of the first nucleic acid fragment and the second nucleic acid fragment; or
the first nucleic acid fragment is linked to the third nucleic acid fragment, and the second nucleic acid fragment is not linked to each of the first nucleic acid fragment and the third nucleic acid fragment; or
the second nucleic acid fragment is linked to the third nucleic acid fragment, and the first nucleic acid fragment is not linked to each of the second nucleic acid fragment and the third nucleic acid fragment; or
the 3' end of the first nucleic acid fragment is linked to the 5' end of the second nucleic acid fragment, and the third nucleic acid fragment is not linked to each of the first nucleic acid fragment and the second nucleic acid fragment; or
the 3' end of the second nucleic acid fragment is linked to the 5' end of the first nucleic acid fragment, and the third nucleic acid fragment is not linked to each of the first nucleic acid fragment and the second nucleic acid fragment; or
the 3' end of the first nucleic acid fragment is linked to the 5' end of the third nucleic acid fragment, and the second nucleic acid fragment is not linked to each of the first nucleic acid fragment and the third nucleic acid fragment; or
the 3' end of the third nucleic acid fragment is linked to the 5' end of the first nucleic acid fragment, and the second nucleic acid fragment is not linked to each of the first nucleic acid fragment and the third nucleic acid fragment; or
the 3' end of the second nucleic acid fragment is linked to the 5' end of the third nucleic acid fragment, and the first nucleic acid fragment is not linked to each of the second nucleic acid fragment and the third nucleic acid fragment; or
the 3' end of the third nucleic acid fragment is linked to the 5' end of the second nucleic acid fragment, and the first nucleic acid fragment is not linked to each of the second nucleic acid fragment and the third nucleic acid fragment; or
the 3' end of the first nucleic acid fragment is linked to the 5' end of the second nucleic acid fragment, and the 3' end of the second nucleic acid fragment is linked to the 5' end of the third nucleic acid fragment; or
the 3' end of the first nucleic acid fragment is linked to the 5' end of the third nucleic acid fragment, and the 3' end of the third nucleic acid fragment is linked to the 5' end of the second nucleic acid fragment; or
the 3' end of the second nucleic acid fragment is linked to the 5' end of the first nucleic acid fragment, and the 3' end of the first nucleic acid fragment is linked to the 5' end of the third nucleic acid fragment; or
the 3' end of the second nucleic acid fragment is linked to the 5' end of the third nucleic acid fragment, and the 3' end of the third nucleic acid fragment is linked to the 5' end of the first nucleic acid fragment; or
the 3' end of the third nucleic acid fragment is linked to the 5' end of the first nucleic acid fragment, and the 3' end of the first nucleic acid fragment is linked to the 5' end of the second nucleic acid fragment; or
the 3' end of the third nucleic acid fragment is linked to the 5' end of the second nucleic acid fragment, and the 3' end of the second nucleic acid fragment is linked to the 5' end of the first nucleic acid fragment.

14. The pharmaceutical formulation according to claim 1 or the nucleic acid molecule according to claim 12, wherein the M protein has an amino acid sequence with at least 89% homology to the amino acid sequence as set forth in SEQ ID NO: 1.

15. The pharmaceutical formulation or the nucleic acid molecule according to claim 14, wherein the M protein has the amino acid sequence with at least 89% homology to the amino acid sequence as set forth in SEQ ID NO: 1, wherein:
an amino acid at site 90 is Y;
an amino acid at site 102 is V;
an amino acid at site 120 is I;
an amino acid at site 144 is A; and
an amino acid at site 180 is L.

16. The pharmaceutical formulation or the nucleic acid molecule according to claim 15, wherein the M protein has the amino acid sequence as set forth in SEQ ID NO: 1.

17. The pharmaceutical formulation according to claim 1 or the nucleic acid molecule according to claim 12, wherein the N protein has an amino acid sequence with at least 91% homology to the amino acid sequence as set forth in SEQ ID NO: 2.

18. The pharmaceutical formulation or the nucleic acid molecule according to claim 17, wherein the N protein has the amino acid sequence as set forth in SEQ ID NO: 2.

19. The pharmaceutical formulation according to claim 1 or the nucleic acid molecule according to claim 12, wherein the S protein has an amino acid sequence with at least 45% homology to the amino acid sequence as set forth in SEQ ID NO: 3.

20. The pharmaceutical formulation or the nucleic acid molecule according to claim 19, wherein the S protein has the amino acid sequence with at least 45% homology to the amino acid sequence as set forth in SEQ ID NO: 3, wherein:
an amino acid at site 515 is V;
an amino acid at site 577 is Q;
an amino acid at site 1385 is V;
an amino acid at site 1386 is V;
an amino acid at site 1397 is F; and
an amino acid at site 1415 is I.

21. The pharmaceutical formulation or the nucleic acid molecule according to claim 20, wherein the S protein has the amino acid sequence as set forth in SEQ ID NO: 3.

22. The pharmaceutical formulation according to claim 1 or the nucleic acid molecule according to claim 12, wherein the S_ec protein has an amino acid sequence with at least 43% homology to amino acids at sites 1 to 1374 of the amino acid sequence as set forth in SEQ ID NO: 3.

23. The pharmaceutical formulation or the nucleic acid molecule according to claim 22, wherein the S_ec protein has the amino acid sequence with at least 43% homology to the amino acids at sites 1 to 1374 of the amino acid sequence as set forth in SEQ ID NO: 3, wherein an amino acid at site 515 is V, and wherein an amino acid at site 577 is Q.

24. The pharmaceutical formulation or the nucleic acid molecule according to claim 23, wherein the S_ec protein has an amino acid sequence of sites 1 to 1374 of SEQ ID NO: 3.

25. The pharmaceutical formulation according to claim 1 or the nucleic acid molecule according to claim 12, wherein the SII protein has an amino acid sequence with at least 62% homology to amino acids at sites 782 to 1433 of the amino acid sequence as set forth in SEQ ID NO: 3.

26. The pharmaceutical formulation or the nucleic acid molecule according to claim 25, wherein the SII protein has the amino acid sequence with at least 62% homology to amino acids at sites 782 to 1433 of the amino acid sequence as set forth in SEQ ID NO: 3, wherein:
an amino acid at site 1385 is V;
an amino acid at site 1386 is V;
an amino acid at site 1397 is F; and
an amino acid at site 1415 is I.

27. The pharmaceutical formulation or the nucleic acid molecule according to claim 26, wherein the SII protein has an amino acid sequence of sites 782 to 1433 of SEQ ID NO: 3.

28. The pharmaceutical formulation according to claim 1 or the nucleic acid molecule according to claim 12, wherein the first nucleic acid fragment has a nucleotide sequence with at least 67% homology to the sequence as set forth in any one of SEQ ID NO: 4 to SEQ ID NO: 7.

29. The pharmaceutical formulation or the nucleic acid molecule according to claim 28, wherein the first nucleic acid fragment has the nucleotide sequence as set forth in any one of SEQ ID NO: 4 to SEQ ID NO: 7.

30. The pharmaceutical formulation according to claim 1 or the nucleic acid molecule according to claim 12, wherein the second nucleic acid fragment has a nucleotide sequence with at least 70% homology to the sequence as set forth in any one of SEQ ID NO: 8 to SEQ ID NO: 11.

31. The pharmaceutical formulation or the nucleic acid molecule according to claim 30, wherein the second nucleic acid fragment has a nucleotide sequence as set forth in any one of SEQ ID NO: 8 to SEQ ID NO: 11.

32. The pharmaceutical formulation according to claim 1 or the nucleic acid molecule according to claim 12, wherein the third nucleic acid fragment has a nucleotide sequence with at least 51% homology to nucleotides at sites 1 to 4122 of the sequence as set forth in any one of SEQ ID NO: 12 to SEQ ID NO: 15.

33. The pharmaceutical formulation or the nucleic acid molecule according to claim 32, wherein the third nucleic acid fragment has a nucleotide sequence of sites 1 to 4122 of the sequence as set forth in any one of SEQ ID NO: 12 to SEQ ID NO: 15.

34. The pharmaceutical formulation or the nucleic acid molecule according to claim 33, wherein the third nucleic acid fragment has a nucleotide sequence with at least 57% homology to nucleotides of sites 2344 to 4299 of any one of SEQ ID NO: 12 to SEQ ID NO: 15.

35. The pharmaceutical formulation or the nucleic acid molecule according to claim 34, wherein the third nucleic acid fragment has a nucleotide sequence of sites 2344 to 4299 of any one of SEQ ID NO: 12 to SEQ ID NO: 15.

36. The pharmaceutical formulation or the nucleic acid molecule according to claim 35, wherein the third nucleic acid fragment has a nucleotide sequence with at least 51% homology to the sequence as set forth in any one of SEQ ID NO: 12 to SEQ ID NO: 15.

37. The pharmaceutical formulation or the nucleic acid molecule according to claim 36, wherein the third nucleic acid fragment has a nucleotide sequence as set forth in any one of SEQ ID NO: 12 to SEQ ID NO: 15.

38. The pharmaceutical formulation according to claim 1 or the nucleic acid molecule according to claim 12, further comprising a fourth nucleic acid fragment, wherein the fourth nucleic acid fragment encodes a signal peptide sequence of MHC I or a sequence having a similar function to a signal peptide of MHC I.

39. The pharmaceutical formulation or the nucleic acid molecule according to claim 38, wherein the signal peptide sequence of MHC I comprises no transmembrane region.

40. The pharmaceutical formulation or the nucleic acid molecule according to claim 38, wherein the signal peptide sequence of MHC I has the amino acid sequence as set forth in SEQ ID NO: 16.

41. The pharmaceutical formulation or the nucleic acid molecule according to claim 38, wherein the fourth nucleic acid fragment has the nucleotide sequence as set forth in any one of SEQ ID NO: 17 to SEQ ID NO: 22.

42. The pharmaceutical formulation according to claim 2 or claim 38 or the nucleic acid molecule according to claim 12 or claim 38, wherein the fifth nucleic acid fragment is located at the 5' end of the nucleic acid fragment or the 5' end of the nucleic acid molecule.

43. The pharmaceutical formulation according to claim 1 or the nucleic acid molecule according to claim 12, further comprising a fifth nucleic acid fragment, wherein the fifth nucleic acid fragment encodes an MITD sequence or a sequence having a similar function to MITD.

44. The pharmaceutical formulation or the nucleic acid molecule according to claim 43, wherein the MITD sequence has the amino acid sequence as set forth in SEQ ID NO: 23.

45. The pharmaceutical formulation or the nucleic acid molecule according to claim 38, wherein the fifth nucleic acid fragment has the nucleotide sequence as set forth in any one of SEQ ID NO: 24 to SEQ ID NO: 32.

46. The pharmaceutical formulation according to claim 2 or claim 43 or the nucleic acid molecule according to claim 12 or claim 43, wherein the fifth nucleic acid fragment is located at the 3' end of the nucleic acid fragment or the 3' end of the nucleic acid molecule.

47. The pharmaceutical formulation according to claim 1 or the nucleic acid molecule according to claim 12, wherein the nucleic acid fragment or the nucleic acid molecule is a linear molecule.

48. The pharmaceutical formulation or the nucleic acid molecule according to claim 47, wherein the nucleic acid fragment or nucleic acid molecule has a nucleotide sequence shown in Table 4.

49. The pharmaceutical formulation according to claim 1, further comprising a pharmaceutical carrier, wherein the pharmaceutical carrier comprises at least one of a liposome, an exosome, a polymer carrier, a viral vector, or a nanoparticle.

50. An expression vector, carrying the nucleic acid molecule according to any one of claims 12 to 48.

51. The expression vector according to claim 50, being a non-viral vector.

52. A recombinant virus, carrying the nucleic acid molecule according to any one of claims 12 to 48.

53. A liposome, comprising a liposome carrier and a nucleic acid fragment, wherein the nucleic acid fragment is as defined in any one of claims 1 to 7 or claims 13 to 48.

54. A vaccine, comprising the pharmaceutical formulation according to any one of claims 1 to 7 or claims 13 to 49, the nucleic acid molecule according to any one of claims 12 to 48, the expression vector according to any one of claims 50 and 51, the recombinant virus according to claim 52, or the liposome according to claim 53.

55. The vaccine according to claim 54, further comprising an adjuvant.

56. The vaccine according to claim 55, wherein the adjuvant comprises at least one of a TLR agonist or Mn²⁺.

57. The vaccine according to claim 56, wherein the TLR agonist comprises at least one of CpG, R837, MPLA, or derivatives thereof.

58. A recombinant cell, carrying a nucleic acid fragment, the nucleic acid molecule according to any one of claims 12 to 48, the expression vector according to any one of claims 50 and 51, or the recombinant virus according to claim 52,
wherein the nucleic acid fragment comprises at least one of a first nucleic acid fragment, a second nucleic acid fragment, or a third nucleic acid fragment; wherein:
the first nucleic acid fragment encodes an M protein of feline infectious peritonitis virus;
the second nucleic acid fragment encodes an N protein of feline infectious peritonitis virus;
the third nucleic acid fragment encodes an S protein, S_ec protein, or SII protein of feline infectious peritonitis virus; and
the first nucleic acid fragment, the second nucleic acid fragment, and the third nucleic acid fragment are linked or not linked.

59. A method for constructing a feline infectious peritonitis virus vaccine, the method comprising:
introducing a nucleic acid fragment, the nucleic acid molecule according to any one of claims 12 to 48, the expression vector according to any one of claims 50 and 51, or the recombinant virus according to claim 52 into a recipient cell,
wherein the nucleic acid fragment comprises at least one of a first nucleic acid fragment, a second nucleic acid fragment, and a third nucleic acid fragment, wherein:
the first nucleic acid fragment encodes an M protein of feline infectious peritonitis virus;
the second nucleic acid fragment encodes an N protein of feline infectious peritonitis virus;
the third nucleic acid fragment encodes an S protein, S_ec protein, or SII protein of feline infectious peritonitis virus; and
the first nucleic acid fragment, the second nucleic acid fragment, and the third nucleic acid fragment are linked or not linked.

60. The method according to claim 59, further comprising, prior to said introducing into the recipient cell:
encapsulating the nucleic acid, the expression vector, or the recombinant virus with an encapsulation carrier.

61. The method according to claim 60, wherein the encapsulation carrier is selected from at least one of a liposome, an exosome, a polymer carrier, a viral vector, or a nanoparticle.

62. The method according to claim 61, wherein the encapsulation carrier is a liposome.

63. The method according to claim 59, wherein the recipient cell is a CRFK cell, HEK293FT cell, HEK293T cell, a BHK cell, or an insect cell.

64. The method according to claim 63, wherein the recipient cell is a CRFK cell.

65. Use of the pharmaceutical formulation according to any one of claims 1 to 7 and claims 13 to 49, the nucleic acid molecule according to any one of claims 12 to 48, the expression vector according to any one of claims 50 and 51, the recombinant virus according to claim 52, or the liposome according to claim 53 in the manufacture of a medicament or vaccine for preventing or treating a disease related to feline infectious peritonitis virus infection.

66. A method for preventing or treating a disease caused by feline infectious peritonitis virus infection, comprising administering to a subject the pharmaceutical formulation according to any one of claims 1 to 7 and claims 13 to 49, the nucleic acid molecule according to any one of claims 12 to 48, the expression vector according to any one of claims 50 and 51, the recombinant virus according to claim 52, the liposome according to claim 53, the vaccine according to any one of claims 54 to 57, or the recombinant cell according to claim 58.

67. The method according to claim 66, wherein the subject is selected from a cat.

68. Use of the pharmaceutical formulation according to any one of claims 1 to 7 and claims 13 to 49, the nucleic acid molecule according to any one of claims 12 to 48, the expression vector according to any one of claims 50 and 51, the recombinant virus according to claim 52, the liposome according to claim 53, the vaccine according to any one of claims 54 to 57, or the recombinant cell according to claim 58 in the prevention or treatment of a disease caused by feline infectious peritonitis virus infection.
